# EUROPEAN PATENT APPLICATION

(11) **EP 4 691 352 A1**
(43) Date of publication of application: **11.02.2026**
(21) Application number: 24876325.2
(22) Date of filing: 06.09.2024
(51) Int. Cl.: A61B 5/0205, A61B 5/1455, A61B 5/332, A61B 5/00

(54) **MEASUREMENT APPARATUS, ELECTRONIC DEVICE, AND CONTROL METHOD THEREOF**

(30) Priority: 13.10.2023 CN 202311331851
(71) Applicant: HUAWEI TECHNOLOGIES CO., LTD., Shenzhen 518129 (CN)
(72) Inventor: CAI, Licheng, Shenzhen, Guangdong, 518129 (CN); CUI, Zhiwei, Shenzhen, Guangdong, 518129 (CN); DENG, Shuang, Shenzhen, Guangdong, 518129 (CN); NIE, Yu, Shenzhen, Guangdong, 518129 (CN)
(74) Representative: Grünecker Patent- und Rechtsanwälte PartG mbB
(86) International application number: PCT/CN2024/117509
(87) International publication number: WO 2025/077505

(57) **Abstract**

Embodiments of this application relate to the field of data monitoring technologies, and provide a monitoring apparatus, an electronic device, and a control method therefor, to resolve a problem of large size of a device having a health sign monitoring function. The monitoring apparatus includes a first circuit board, a light emitting device, an optical-to-electrical converter, a metal cover plate, and a light transmission structure. The optical-to-electrical converter and the light emitting device are disposed on a same side of the first circuit board, and are electrically connected to the first circuit board. The metal cover plate covers the first circuit board, and the metal cover plate is electrically connected to the first circuit board. A first light through hole exposing the light emitting device and a second light through hole exposing the optical-to-electrical converter are disposed on the metal cover plate. The light transmission structure fills the first light through hole, the second light through hole, and an accommodation cavity, wraps the light emitting device and the optical-to-electrical converter, and is connected to the light emitting device, the optical-to-electrical converter, the first circuit board, and the metal cover plate to form an integrated structural part. This reduces a size of the monitoring apparatus.

## Description

This application claims priority to Chinese Patent Application No. 202311331851.X, filed with the China National Intellectual Property Administration on October 13, 2023 and entitled "MONITORING APPARATUS, ELECTRONIC DEVICE, AND CONTROL METHOD THEREFOR ", which is incorporated herein by reference in its entirety.

### TECHNICAL FIELD

This application relates to the field of data monitoring technologies, and in particular, to a monitoring apparatus, an electronic device, and a control method therefor.

### BACKGROUND

As the living standard is continuously improved, users have higher requirements on functions of electronic devices. A device integrated with a human health sign monitoring function can monitor health sign data of the user in a timely manner, for example, monitor a heart rate, blood oxygen, an electrocardiogram, and blood pressure, so that the user can learn about physical conditions of the user at any time, to prevent diseases. However, currently, a device having the foregoing health sign monitoring function has a large size, which is not conducive to a development trend of component miniaturization and portability.

### SUMMARY

This application provides a monitoring apparatus, an electronic device, and a control method therefor, to resolve a problem of large size of a device having a health sign monitoring function.

To achieve the foregoing objective, this application uses the following technical solutions.

According to an aspect of this application, a monitoring apparatus is provided. The monitoring apparatus includes a first circuit board, a light emitting device, an optical-to-electrical converter, a metal cover plate, and a light transmission structure. The light emitting device is disposed on the first circuit board, and the light emitting device is electrically connected to the first circuit board. The optical-to-electrical converter and light emitting device are disposed on a same side of the first circuit board, and the optical-to-electrical converter is electrically connected to the first circuit board. In addition, the metal cover plate covers the first circuit board, and the metal cover plate is electrically connected to the first circuit board. An accommodation cavity is formed between the metal cover plate and the first circuit board, and the light emitting device and the optical-to-electrical converter are disposed in the accommodation cavity. A first light through hole and a second light through hole that penetrate the metal cover plate are disposed on the metal cover plate. The first light through hole exposes a light exit surface of the light emitting device, and the second light through hole exposes a light receiving surface of the optical-to-electrical converter. The light transmission structure fills the first light through hole, the second light through hole, and the accommodation cavity. The light transmission structure wraps the light emitting device and the optical-to-electrical converter, and is connected to the light emitting device, the optical-to-electrical converter, the first circuit board, and the metal cover plate to form an integrated structural part.

In conclusion, in the monitoring apparatus provided in this embodiment of this application, the light emitting device and the optical-to-electrical converter are covered by the metal cover plate, and the light transmission structure may cover the light exit surface of the light emitting device and the light receiving surface of the optical-to-electrical converter, to ensure that the light emitting device and the optical-to-electrical converter can work normally. In addition, the light transmission structure may further cover a side surface of the light emitting device and the optical-to-electrical converter. Therefore, surfaces of the light emitting device and the optical-to-electrical converter other than surfaces facing the first circuit board may be covered by the light transmission structure. In this way, the light transmission structure can wrap the light emitting device and the optical-to-electrical converter, so that the light transmission structure can be connected to the light emitting device, the optical-to-electrical converter, the first circuit board, and the metal cover plate to form the integrated structural part. Based on this, the monitoring apparatus provided in this embodiment of this application can connect the metal cover plate used as an ECG electrode to the light emitting device and the optical-to-electrical converter through the light transmission structure embedded in the metal cover plate. Therefore, no bracket configured to cover the light emitting device and the optical-to-electrical converter and no adhesive layer and transparent plate stacked on the bracket need to be disposed in the monitoring apparatus. In this way, size requirements of the adhesive layer and the transparent plate do not need to be considered for a length, a width, and a height of the monitoring apparatus. Therefore, a size of the monitoring apparatus can be reduced, so that a miniaturization design of the monitoring apparatus can be implemented. In this case, when the monitoring apparatus with the miniaturization design is used in the electronic device, a size of the electronic device can be reduced, so that a problem of large size of a device having a health sign monitoring function can be alleviated.

In an optional implementation, the monitoring apparatus includes a first lever, a deformation plate, and a pressure-sensitive element. The first lever is disposed on a side that is of the first circuit board and that is away from the metal cover plate, and a first end of the first lever abuts against or is connected to the first circuit board. The deformation plate is disposed on a side that is of the first lever and that is away from the first circuit board, the deformation plate has a first cantilever end and a fastening end, the first cantilever end abuts against or is connected to a second end of the first lever, and the fastening end is configured to fasten the deformation plate. The pressure-sensitive element covers at least a part of the first cantilever end, and the pressure-sensitive element is electrically connected to the first circuit board. In this way, in a process in which a finger of a user presses down the metal cover plate, an external force applied by the user may be transmitted to the first circuit board through the metal cover plate, and then transmitted to the first lever located below the first circuit board through the first circuit board. Because the first lever abuts against or is connected to the first cantilever end of the deformation plate, and the fastening end of the deformation plate fastens the deformation plate to a component, for example, a middle frame, the first cantilever end of the deformation plate may deform downward under pressing action of the first lever. In addition, because the pressure-sensitive element covers at least the part of the first cantilever end, the pressure-sensitive element may convert deformation of the first cantilever end into an electrical signal by using a piezoelectric sensing principle, and transmit the electrical signal to the first circuit board that is electrically connected to the pressure-sensitive element, to monitor a pressing force of the user.

In an optional implementation, the pressure-sensitive element is disposed on a side that is of the deformation plate and that faces the metal cover plate. The monitoring apparatus further includes a mounting pillar. The mounting pillar is located on a side that is of the deformation plate and that is away from the metal cover plate. A first end of the mounting pillar is connected to the fastening end, and a second end of the mounting pillar is configured to fasten the monitoring apparatus. For example, the entire monitoring apparatus may be fastened to the middle frame through the second end of the mounting pillar. In this way, because the pressure-sensitive element is disposed on the side that is of the deformation plate and that faces the metal cover plate, a disposing position of the mounting pillar is not affected. In addition, because the first end of the mounting pillar is connected to the fastening end of the deformation plate, the fastening end of the deformation plate is fastened.

In an optional implementation, the pressure-sensitive element includes a resistance strain gauge, and a part that is of the resistance strain gauge and that covers the first cantilever end has a first resistor and a second resistor. The monitoring apparatus further includes a third resistor and a fourth resistor. The first resistor and the second resistor are connected in series to form a first circuit. The third resistor and the fourth resistor are connected in series to form a second circuit. The first circuit and the second circuit are connected in parallel to form a first full-bridge circuit. The first full-bridge circuit is electrically connected to the first circuit board. In this way, when the user presses the metal cover plate of the monitoring apparatus, the first cantilever end of the deformation plate deforms. In this case, resistance values of the first resistor and the second resistor in the pressure-sensitive element that covers the first cantilever end change, so that a voltage output by the first full-bridge circuit changes. The voltage matches a pressure at which the user presses the metal cover plate, so that a user pressing pressure, for example, a first pressure value, can be monitored by obtaining a voltage difference.

In an optional implementation, the deformation plate further has a second cantilever end, and the fastening end is located between the first cantilever end and the second cantilever end. The pressure-sensitive element covers at least a part of the second cantilever end. The monitoring apparatus further includes a second lever, the second lever is disposed on the side that is of the first circuit board and that is away from the metal cover plate, a first end of the second lever abuts against or is connected to the first circuit board, and a second end of the second lever abuts against or is connected to the second cantilever end. In this way, in the process in which the finger of the user presses the metal cover plate downward, the external force applied by the user may be transmitted to the first circuit board through the metal cover plate, and then transmitted to the first lever and the second lever that are located below the first circuit board through the first circuit board. Because the first lever and the second lever respectively abut against or are connected to the first cantilever end and the second cantilever end of the deformation plate, and the fastening end that is of the deformation plate and that is located between the first cantilever end and the second cantilever end fastens the deformation plate to a component, for example, a middle frame, the first cantilever end and the second cantilever end of the deformation plate may deform downward under pressing action of the first lever and the second lever. In addition, because the pressure-sensitive element covers at least the part of the first cantilever end and at least the part of the second cantilever end, the pressure-sensitive element may convert deformation of the first cantilever end and the second cantilever end into an electrical signal by using a piezoelectric sensing principle, and transmit the electrical signal to the first circuit board electrically connected to the pressure-sensitive element, to monitor a pressing force of the user. It can be learned from the foregoing that when the user presses the metal cover plate of the monitoring apparatus, the processor of the electronic device may compare a deformation amount of the first cantilever end and a deformation amount of the second cantilever end that are from the deformation plate, to determine whether a pressing position of the user is at a middle position of the metal cover plate, to improve monitoring precision. Therefore, the foregoing monitoring apparatus may be referred to as a dual-channel monitoring structure.

In an optional implementation, the deformation plate further has a second cantilever end, the second cantilever end is located on a side that is of the first cantilever end and that is away from the fastening end, and there is a gap between the first cantilever end and the second cantilever end. The pressure-sensitive element covers at least a part of the second cantilever end. The second end of the first lever further abuts against or is connected to the second cantilever end. In this way, one lever, that is, the first lever, is disposed, so that the first cantilever end and the second cantilever end can be pressed at the same time, to drive the first cantilever end and the second cantilever end to deform at the same time, to implement dual-channel pressure monitoring.

In an optional implementation, the pressure-sensitive element includes the resistance strain gauge. A part that is of the resistance strain gauge and that covers the second cantilever end has a fifth resistor and a sixth resistor. The fifth resistor and the sixth resistor are connected in series to form a third circuit, the second circuit and the third circuit are connected in parallel to form a second full-bridge circuit, and the second full-bridge circuit is electrically connected to the first circuit board. In this way, when the user presses the metal cover plate of the monitoring apparatus, the second cantilever end of the deformation plate deforms. In this case, resistance values of the fifth resistor and the sixth resistor in the pressure-sensitive element that covers the second cantilever end change, so that a voltage output by the second full-bridge circuit changes. The voltage matches a pressure at which the user presses the metal cover plate, so that, a user pressing pressure, for example, a second pressure value, can be monitored by obtaining a voltage difference. It can be learned from the foregoing that when the user presses the metal cover plate of the monitoring apparatus, the user pressing pressure detected by the first full-bridge circuit, for example, the first pressure value, comes from the deformation amount of the first cantilever end of the deformation plate, and the user pressing pressure detected by the second full-bridge circuit, for example, the second pressure value, comes from the deformation amount of the second cantilever end of the deformation plate. The first cantilever end and the second cantilever end are respectively located on two sides of the fastening end. Therefore, the processor of the electronic device may determine, by comparing a difference between the first pressure value and the second pressure value, whether a pressing position of the user is in a middle position of the metal cover plate, to improve monitoring precision. Therefore, the foregoing monitoring apparatus may be referred to as a dual-channel monitoring structure.

In an optional implementation, the first circuit board includes a flexible circuit board and a supportive plate. The supportive plate is disposed on a side that is of the flexible circuit board and that is away from the metal cover plate, and the supportive plate is connected to or abuts against the first lever. Rigidity of the supportive plate is greater than rigidity of the flexible circuit board, so that the supportive plate can perform supporting. In addition, when the user presses the metal cover plate, a force transmitted to the first circuit board may be transmitted to the first lever through the supportive plate. Alternatively, the first circuit board includes a printed circuit board. When the user presses the metal cover plate, a force transmitted to the first circuit board may be transmitted to the first lever.

In an optional implementation, the monitoring apparatus further includes a first metal electrode plate, the first metal electrode plate is disposed on a side that is of the supportive plate and that is away from the metal cover plate, and the first metal electrode plate is electrically connected to the flexible circuit board. The pressure-sensitive element is located between the deformation plate and the metal supportive plate, the pressure-sensitive element includes a second metal electrode plate, and a capacitor is formed between the second metal electrode plate and the first metal electrode plate. In this way, when the user presses the metal cover plate of the monitoring apparatus, a spacing between the foregoing two metal electrodes changes, so that a capacitance value of the capacitor can be changed. The capacitance value matches the pressing pressure of the user, so that an objective of monitoring the pressing pressure of the user can be achieved.

In an optional implementation, the supportive plate includes a metal material. The pressure-sensitive element is located between the deformation plate and the supportive plate, and the pressure-sensitive element includes a metal coil. In this case, a current may be supplied to the coil. When the user presses the metal cover plate of the monitoring apparatus, a spacing between the supportive plate and the pressure-sensitive element changes, so that a magnetic field generated by the coil can be changed, and impedance of the coil changes. The impedance may match a pressing force of the user, to monitor the pressing force of the user.

In an optional implementation, the monitoring apparatus further includes conductive silicone gel. The conductive silicone gel is disposed between the metal cover plate and the first circuit board. A first end of the conductive silicone gel is electrically connected to the metal cover plate, and a second end of the conductive silicone gel is electrically connected to the first circuit board. In this case, the two ends of the conductive silicone gel may be separately welded to pads of the metal cover plate and the first circuit board by using a welding process, so that the metal cover plate may be electrically connected to the first circuit board through the conductive silicone gel, to implement signal interconnection between the conductive silicone gel and the first circuit board.

In an optional implementation, the monitoring apparatus further includes conductive adhesive. The conductive adhesive is disposed between the metal cover plate and the first circuit board. The conductive adhesive is bonded to the metal cover plate and a copper exposure part of the first circuit board, and the conductive adhesive is electrically connected to the metal cover plate and the first circuit board. In this case, a part that is of the first circuit board and that is electrically connected to the conductive adhesive may not need to be provided with a pad. The conductive adhesive is bonded to the copper exposure part of the first circuit board, and a side that is of the conductive adhesive and that is away from the copper exposure part may be bonded to the metal cover plate, so that the metal cover plate may implement signal interconnection with the first circuit board through the conductive adhesive.

In an optional implementation, the monitoring apparatus further includes a metal dome. The metal dome is disposed in the accommodation cavity. A first end of the metal dome is welded to the metal cover plate, and a second end of the metal dome abuts against a copper exposure part of the first circuit board. In a process in which the second end of the metal dome abuts against the copper exposure part of the first circuit board, the metal dome may elastically deform, and the deformed metal dome may be in closer contact with the copper exposure part of the first circuit board. Alternatively, the second end of the metal dome is welded to the first circuit board, and the first end of the metal dome abuts against the metal cover plate. Similarly, in a process in which the first end of the metal dome abuts against the metal cover plate, the metal dome may elastically deform, and the deformed metal dome may be in closer contact with the metal cover plate. In this way, the metal cover plate may implement signal interconnection with the first circuit board through the metal dome.

In an optional implementation, the metal cover plate includes a metal cover plate body and a light shielding blocking plate. The metal cover plate body covers the first circuit board, and the metal cover plate body is electrically connected to the first circuit board. The first light through hole and the second light through hole that are spaced from each other are disposed on the metal cover plate body. The light shielding blocking plate is disposed in the accommodation cavity, and the light shielding blocking plate is connected to the metal cover plate body and the first circuit board. The light shielding blocking plate is located between the first light through hole and the second light through hole, and separates the light emitting device from the optical-to-electrical converter. In this way, because the light shielding blocking plate is prepared by using a metal material, the light shielding blocking plate has a light shielding performance. Therefore, disposing the light shielding blocking plate between the light emitting device and the optical-to-electrical converter can reduce or even avoid direct incidence of light emitted by the light emitting device on the optical-to-electrical converter. This can reduce optical crosstalk between the light emitting device and the optical-to-electrical converter, and improve monitoring precision of the optical-to-electrical converter.

In an optional implementation, the monitoring apparatus may further include a light shielding connection layer, the light shielding connection layer is disposed between the metal cover plate and the first circuit board, and the light shielding connection layer is connected to the metal cover plate and the first circuit board. In this way, a bottom of the metal cover plate may be bonded to the first circuit board through the light shielding connection layer, to reduce or avoid light leakage of the light emitted by the light emitting device from the bottom of the metal cover plate, and improve light utilization.

In an optional implementation, a surface that is of the light transmission structure and that is away from the first circuit board and a surface that is of the metal cover plate and that is away from the first circuit board are spliced into an arc surface. In this way, the surface that is of the light transmission structure and that is away from the first circuit board may smoothly transit at a junction position of the surface that is of the metal cover plate and that is away from the first circuit board, to improve pressing comfort of a user.

In an optional implementation, the monitoring apparatus includes a metal coating, and the metal coating covers the surface of the metal cover plate that is away from the first circuit board. In this way, a material of the metal coating may be used for appearance decoration. In addition, the metal cover plate and the metal coating may have a same conductivity or different conductivities. In this case, the ECG electrode may include the metal cover plate and the metal coating.

In an optional implementation, the light emitting device includes a first light emitting component, the first light emitting component is configured to emit at least one of red light or infrared light, and there is a first spacing d1 between the first light emitting component and the optical-to-electrical converter. The red light emitted by the first light emitting component and the infrared light emitted by the second light emitting component have long wavelengths, and can reach subcutaneous tissue, to monitor a blood volume in an artery. The red light is mainly absorbed by oxyhemoglobin in the blood, and the infrared light is mainly absorbed by hemoglobin and deoxygenated hemoglobin in blood. Therefore, the processor may calculate blood oxygen saturation by measuring a proportion of hemoglobin combined with oxygen in the blood based on an absorption difference between the red light and the infrared light collected by the optical-to-electrical converter 23, to obtain blood oxygen data. In addition, a second light emitting component is configured to emit at least one of yellow light, green light, or blue light, there is a second spacing d2 between the second light emitting component and the optical-to-electrical converter, and d1>d2. The blue light or the green light emitted by the second light emitting component has a short wavelength, and can reach a surface layer of the skin, to monitor a blood volume in a capillary. The yellow light emitted by the second light emitting component can reach the dermis layer, so that a blood volume of blood in a small artery can be monitored.

In an optional implementation, when the first spacing d1 falls within a range 3.5 mm≤d1≤5.5 mm, light emitted by the first light emitting component can be reflected by the skin of the user, and can be more fully and easily received by the optical-to-electrical converter. Therefore, a signal collected by the optical-to-electrical converter can be more accurate, and PPG data obtained by the electronic device can be more accurate. When the second spacing d2 falls within a range 3.0 mm≤d1≤4.5 mm, the PPG data obtained by the electronic device may be more accurate. In an optional implementation, the light transmission structure includes a resin material, and the first circuit board, the light emitting device, the optical-to-electrical converter, the metal cover plate, and the light transmission structure are an injection-molded integrated structural part. In this way, the injection-molded integrated structural part may be formed by using a one-time processing technology, for example, an injection molding technology. The injection-molded integrated structural part may include the light emitting device, the optical-to-electrical converter, the first circuit board, the metal cover plate, and the light transmission structure of the monitoring apparatus. The light transmission structure not only may connect the light emitting device and the optical-to-electrical converter to the metal cover plate and the first circuit board, but also may wrap a surface and a periphery of the light emitting device and the optical-to-electrical converter. Therefore, the light emitting device and the optical-to-electrical converter may be packaged, to reduce impact of external water vapor and impurities on performance of the light emitting device and the optical-to-electrical converter, and improve waterproof and dustproof reliability of the entire monitoring apparatus.

In an optional implementation, the monitoring apparatus includes a plurality of protrusion structures disposed on the surface that is of the metal cover plate and that is away from the first circuit board. In a pressing process, the user may clearly feel a concave-convex feeling of a touch surface. A position of the protrusion structure may match a preset pressing position, to guide the user to press the preset position, thereby improving monitoring precision.

According to another aspect of the embodiments of this application, an electronic device is provided. The electronic device may include a middle frame and any one of the foregoing monitoring apparatuses. The monitoring apparatus is connected to the middle frame, and mounting groove is disposed on an outer surface of the middle frame. The first circuit board, the light emitting device, the optical-to-electrical converter, the metal cover plate, and the light transmission structure are located in the mounting groove, and the first circuit board is closer to a bottom of the mounting groove relative to the metal cover plate. The electronic device has same technical effect as the monitoring apparatus provided in the foregoing embodiment. Details are not described herein again.

In an optional implementation, a first mounting hole that penetrates the middle frame is disposed at the bottom of the mounting groove. The monitoring apparatus includes a deformation plate and a mounting pillar. In addition, the electronic device includes an elastic layer and a first clamping ring. The deformation plate is located in the mounting groove, and the deformation plate has a fastening end. The mounting pillar is located on a side that is of the deformation plate and that is away from the metal cover plate, and penetrates the first mounting hole. A first end of the mounting pillar is connected to the fastening end. A second end of the mounting pillar extends out of the first mounting hole and is located in the middle frame. In addition, the elastic layer is located in the mounting groove, and the elastic layer is disposed between the deformation plate and the bottom of the mounting groove. The elastic layer can reduce a probability of plastic deformation of the deformation plate under pressing action of the user. The first clamping ring is located in the middle frame, the first clamping ring is clamped to the second end of the mounting pillar, and the first clamping ring abuts against the middle frame. In this way, in a process in which the user presses the monitoring apparatus, the first clamping ring may limit movement of the mounting pillar in a vertical direction.

In an optional implementation, the elastic layer includes silicone gel or rubber. A modulus of the silicone gel or the rubber may increase as compression deformation increases. In this way, when the user applies large pressing pressure, a modulus of the elastic layer increases after the elastic layer is pressed, to support the deformation plate, thereby implementing a limiting function, so that the first cantilever end and the second cantilever end of the deformation plate are not easy to plastically deform. Alternatively, the elastic layer includes a soft rubber layer and a plurality of particles doped in the soft rubber layer. Hardness of the particle is greater than hardness of the soft rubber layer. The soft rubber layer includes silicone gel or rubber, and the particle includes at least one of metal, plastic, or rubber. In this way, under the pressing pressure of the user, the soft rubber layer is pressed to undergo elastic deformation, so that a spacing between the particles is reduced, and further, at least some particles directly contact each other. Therefore, rigidity of the entire elastic layer is increased, the deformation plate can be supported, thereby achieving a limiting effect, so that the first cantilever end and the second cantilever end of the deformation plate are not easy to plastically deform.

In an optional implementation, a first sealing groove is disposed on a part that is of the mounting pillar and that is located in the first mounting hole. The electronic device includes a first sealing ring, the first sealing ring is located in the first sealing groove, and the first sealing ring abuts against a hole wall of the first mounting hole and a groove wall of the first sealing groove. The first sealing ring seals a gap between the first mounting hole and the mounting pillar, to reduce a probability that external water vapor enters the middle frame through the mounting groove and the first mounting hole, and improve product reliability.

In an optional implementation, the first mounting hole that penetrates the middle frame is disposed at the bottom of the mounting groove, and the monitoring apparatus includes a first lever. In addition, the electronic device may further include a first bonding layer and a second clamping ring. The first lever penetrates the first mounting hole, the first lever is disposed on a side that is of the first circuit board and that is away from the metal cover plate, a first end of the first lever abuts against or is connected to the first circuit board, and a second end of the first lever extends out of the first mounting hole and is located in the middle frame. In addition, the first bonding layer is located in the mounting groove, the first bonding layer is disposed between the first circuit board and the bottom of the mounting groove, and the first bonding layer is connected to the first circuit board and the bottom of the mounting groove. The second clamping ring is located in the middle frame, the second clamping ring is clamped to the second end of the first lever, and the second clamping ring abuts against the middle frame. In this way, in the process in which the user presses the monitoring apparatus, the second clamping ring may limit movement of the first lever in the vertical direction.

In an optional implementation, the monitoring apparatus further includes a deformation plate, the deformation plate is located in the middle frame, and the deformation plate has a fastening end. In addition, the electronic device further includes a threaded connector. The threaded connector is located in the middle frame, the threaded connector penetrates the fastening end of the deformation plate, and is connected to the middle frame, to fasten the deformation plate.

In an optional implementation, a first mounting hole and a second mounting hole that penetrate the middle frame are disposed at the bottom of the mounting groove. In addition, the monitoring apparatus includes a first lever and a second lever. The first lever penetrates the first mounting hole, the first lever is disposed on a side that is of the first circuit board and that is away from the metal cover plate, a first end of the first lever abuts against or is connected to the first circuit board, and a second end of the first lever extends out of the first mounting hole and is located in the middle frame. The second lever penetrates the second mounting hole, the second lever is disposed on a side that is of the first circuit board and that is away from the metal cover plate, a first end of the second lever abuts against or is connected to the first circuit board, and a second end of the second lever extends out of the second mounting hole and is located in the middle frame. In addition, the electronic device further includes a first spring and a second spring. The first spring is located in the mounting groove, a part of the first lever penetrates the first spring, and two ends of the first spring abut against the first lever and a groove bottom of the mounting groove respectively. The second spring is located in the mounting groove, a part of the second lever penetrates the second spring, and two ends of the second spring abut against the second lever and the groove bottom of the mounting groove respectively. When the user does not press the monitoring apparatus, the first spring and the second spring may be in a compressed state, and after the user cancels a pressing force applied to the metal cover plate, the deformation plate that deforms can be restored to an initial position.

In an optional implementation, the first circuit board includes a flexible circuit board, and a third mounting hole that penetrates the middle frame is disposed at the bottom of the mounting groove. In addition, the electronic device further includes a cap, a second bonding layer, and a second circuit board. The cap is disposed in the third mounting hole, and blocks the third mounting hole. The second bonding layer is disposed in the third mounting hole, and is located on a side that is of the cap and that is away from the mounting groove. The second bonding layer is bonded to the cap and the middle frame. The second circuit board is disposed in the middle frame. A part of the flexible circuit board penetrates the cap and the second bonding layer, extends into the middle frame, and is electrically connected to the second circuit board. The cap can block the third mounting hole, to implement waterproofing and dustproofing. The second bonding layer may be glue, and the second bonding layer is configured to bond the cap to the middle frame.

According to another aspect of the embodiments of this application, a control method of an electronic device is provided. The electronic device includes a monitoring apparatus. The monitoring apparatus has a button function and a health monitoring function, and the monitoring apparatus includes a light emitting device, an optical-to-electrical converter, a metal cover plate, and a pressure-sensitive element. The control method includes: scanning the pressure-sensitive element at a first scanning frequency to identify a pressing event of a user on the monitoring apparatus. Then, whether the health monitoring function is enabled is determined. If the health monitoring function is enabled, the health monitoring function is performed. If the health monitoring function is not enabled, the button function is performed. The control method of the electronic device has same technical effect as the electronic device provided in the foregoing embodiment. Details are not described herein again.

In an optional implementation, the health monitoring function is specifically displaying a guide image. Then, if a human body of the user directly presses the monitoring apparatus, the pressure-sensitive element is scanned at a second scanning frequency to obtain a pressure value applied by the user to the metal cover plate, the light emitting device and the optical-to-electrical converter are started, and a voltage is applied to the metal cover plate to obtain an electrocardiosignal of the user. The second scanning frequency is greater than the first scanning frequency. Then, if the pressure value is greater than or equal to a first threshold and less than or equal to a second threshold, a monitoring result is determined based on at least one of the pressure value, an output signal of the optical-to-electrical converter, and the electrocardiosignal. Then, the monitoring result is displayed, where the monitoring result includes at least one of a blood oxygen value, a heart rate value, electrocardiogram data, and a blood pressure value, to collect blood oxygen, heart rate, electrocardiogram, and blood pressure data, to complete health monitoring on the user.

### BRIEF DESCRIPTION OF DRAWINGS

FIG. 1A is a diagram of a structure of an electronic device according to an embodiment of this application;
FIG. 1B is a diagram of an exploded structure of the electronic device shown in FIG. 1A;
FIG. 2A is a diagram of a user using the electronic device shown in FIG. 1A according to an embodiment of this application;
FIG. 2B is a diagram of a structure in which a user presses a monitoring apparatus shown in FIG. 1A with a finger;
FIG. 3 is a diagram of a structure of a monitoring apparatus in FIG. 1A;
FIG. 4 is a sectional view obtained by performing sectioning along O1-O2 in FIG. 2B;
FIG. 5 is a diagram of a working scenario of a PPG module according to an embodiment of this application;
FIG. 6 is a diagram of a structure of a PPG module according to an embodiment of this application;
FIG. 7 is another sectional view obtained by performing sectioning along O1-O2 in FIG. 2B;
FIG. 8A is diagram of another structure of a monitoring apparatus in FIG. 1A;
FIG. 8B is still another sectional view obtained by performing sectioning along O1-O2 in FIG. 2B;
FIG. 8C is a diagram of a structure of a metal cover plate in FIG. 8B;
FIG. 9 is a sectional view of a partial structure obtained by performing sectioning along O1-O2 in FIG. 2B;
FIG. 10A is a diagram of an electrical connection structure between a metal cover plate and a first circuit board according to an embodiment of this application;
FIG. 10B is a diagram of another electrical connection structure between a metal cover plate and a first circuit board according to an embodiment of this application;
FIG. 11A is yet another sectional view obtained by performing sectioning along O1-O2 in FIG. 2B;
FIG. 11B is sectional view obtained by performing sectioning along A1-A2 in FIG. 11A;
FIG. 12A is a diagram of a structure of a PPG module according to a related technology;
FIG. 12B is a sectional view obtained by performing sectioning along B1-B2 in FIG. 12A;
FIG. 13 is a diagram of a structure of a monitoring device according to an embodiment of this application;
FIG. 14A is a sectional view of a partial structure obtained by performing sectioning along O1-O2 in FIG. 2B;
FIG. 14B is a diagram of deformation of a deformation plate in FIG. 14A;
FIG. 15 is a diagram of a structure of a monitoring device according to an embodiment of this application;
FIG. 16A is a sectional view of a partial structure obtained by performing sectioning along O1-O2 in FIG. 2B;
FIG. 16B is a sectional view of a partial structure obtained by performing sectioning along 01-02 in FIG. 2B
FIG. 17A is a diagram of a state of an elastic layer in FIG. 16A;
FIG. 17B is a diagram of another state of an elastic layer in FIG. 16A;
FIG. 17C is a diagram of still another state of an elastic layer in FIG. 16A;
FIG. 18A is a diagram of a structure of a pressure-sensitive element according to an embodiment of this application;
FIG. 18B is a diagram of another structure of a pressure-sensitive element according to an embodiment of this application;
FIG. 19A is a diagram of an electrical connection manner of a pressure-sensitive element according to an embodiment of this application;
FIG. 19B is a diagram of a circuit structure of some components in FIG. 19A;
FIG. 19C is a diagram of a circuit structure of some components in FIG. 19A;
FIG. 20 is a sectional view of a partial structure obtained by performing sectioning along O1-O2 in FIG. 2B;
FIG. 21 is a sectional view of a partial structure obtained by performing sectioning along C1-C2 in FIG. 2B;
FIG. 22 is a sectional view of a partial structure obtained by performing sectioning along O1-O2 in FIG. 2B;
FIG. 23A is a diagram of a partial structure of a monitoring apparatus according to an embodiment of this application;
FIG. 23B is a diagram of a partial structure of a monitoring apparatus according to an embodiment of this application;
FIG. 24 is a diagram of a structure of an electronic device according to embodiment of this application;
FIG. 25 is a flowchart of a control method of an electronic device according to an embodiment of this application;
FIG. 26 is a diagram of a display interface of an electronic device according to an embodiment of this application;
FIG. 27 is a diagram of another display interface of an electronic device according to an embodiment of this application;
FIG. 28 is a diagram of specific steps of S107 in FIG. 25;
FIG. 29 is a diagram of still another display interface of an electronic device according to an embodiment of this application; and
FIG. 30 is a diagram of yet another display interface of an electronic device according to an embodiment of this application.

### Reference numerals:

01: electronic device; 10: display; 11: middle frame; 12: rear cover; 20: monitoring apparatus; 21: first circuit board; 22: light emitting device; 23: optical-to-electrical converter; 24: metal cover plate; 25: light transmission structure; 200: accommodation cavity; 201: first light through hole; 202: second light through hole; 100: epidermis layer; 101: dermis layer; 102: subcutaneous tissue; 221: first light emitting component; 222: second light emitting component; 2211: first light source; 2212: second light source; 2221: third light source; 2222: fourth light source; 241: metal coating; 27: light shielding connection layer; 271: avoidance hole; 251: first light transmission structure; 252: second light transmission structure; 243: metal cover plate body; 244: light shielding blocking plate; 30: conductive silicone gel; 31: conductive adhesive; 210: copper exposure part; 32: metal dome; 110: transparent plate; 111: adhesive layer; 112: bracket; 400: pressure sensing module; 41: first lever; 42: second lever; 43: deformation plate; 44: pressure-sensitive element; 430: fastening end; 431: first cantilever end; 432: second cantilever end; 2101: FPC; 2102: supportive plate; 220: injection-molded integrated structural part; 300: mounting groove; 301: first mounting hole; 302: second mounting hole; 51: first clamping ring; 52: first sealing ring; 53: elastic layer; 54: cap; 55: second bonding layer; 501: first sealing groove; 60: second circuit board; 45: first metal electrode plate; 46: second metal electrode plate; 531: soft rubber layer; 532: particle; 61: threaded connector; 71: first spring; 72: second spring; 56: first bonding layer; 57: second clamping ring; 70: pressure scale; 80: health monitoring function button; and 81: prompt dialog box.

### DESCRIPTION OF EMBODIMENTS

The following describes the technical solutions in embodiments of this application with reference to the accompanying drawings in embodiments of this application. It is clear that the described embodiments are merely a part rather than all of embodiments of this application.

Terms such as "first" and "second" below are merely used for ease of description, and shall not be understood as indicating or implying relative importance or implicitly indicating a quantity of indicated technical features. Therefore, a feature limited by "first", "second", or the like may explicitly or implicitly include one or more features. In the descriptions of this application, unless otherwise stated, "a plurality of" means two or more than two.

In this application, unless otherwise clearly specified and limited, a term "connection" should be understood in a broad sense. For example, the "connection" may be a fixed mechanical connection, or may be a detachable mechanical connection or an integrated connection, or the "connection" may be a direct connection, or may be an indirect connection implemented through an intermediate medium.

In addition, unless otherwise specified and limited, the term "electrical connection" should be understood in a broad sense. For example, the "electrical connection" may be a direct electrical connection, for example, physical contact and electrical conduction between two components, or may be understood as that different components in a circuit structure are electrically connected through a physical line that can transmit an electrical signal, for example, a printed circuit board (printed circuit board, PCB) copper foil or a wire, to transmit the electrical signal. Alternatively, the "electrical connection" may be an indirect electrical connection between two components through an intermediate medium. Alternatively, the "electrical connection" may be that two components are electrically connected in a spaced/non-contact manner, for example, the two components are electrically connected through capacitive coupling, to transmit an electrical signal. A "communication connection" may refer to an electrical signal transmission, including a wireless communication connection and a wired communication connection. The wireless communication connection does not require a physical medium and does not belong to a connection relationship that defines a construction of a product.

In embodiments of this application, "perpendicular" and "parallel" are described to respectively represent being roughly vertical and being roughly parallel within an allowed error range. The error range may be a range in which a deviation angle is less than or equal to 5°, 8°, or 10° relative to being absolutely vertical and being absolutely parallel. This is not specifically limited herein.

In embodiments of this application, orientation terms such as "upper", "lower", "left", and "right" may include but are not limited to definitions based on illustrated orientations in which components in the accompanying drawings are placed. It should be understood that these directional terms may be relative concepts, are used for description and clarification of relative positions, and may vary accordingly depending on a change in the orientations in which the components in the accompanying drawings are placed in the accompanying drawings.

In the accompanying drawings of embodiments of this application, an assembly is represented by using a guide line with an arrow, a component is represented by using only a guide line, and a hollow-out structure like an opening or a hole is represented by using a guide line with a wavy line at an end.

An embodiment of this application provides an electronic device. The electronic device may be applied to various communication systems or communication protocols, for example, a Bluetooth (Bluetooth, BT) communication technology, a global positioning system (global positioning system, GPS) communication technology, a global system of mobile communication (global system of mobile communication, GSM) communication technology, a wireless fidelity (wireless fidelity, Wi-Fi) communication technology, a wideband code division multiple access (wideband code division multiple access wireless, WCDMA) communication technology, long term evolution (long term evolution, LTE), a 5G communication technology, and another future communication technology.

The electronic device in this embodiment of this application may be an intelligent wearable device, for example, a human body health monitoring wearable device that can monitor a health sign parameter (for example, at least one of a heart rate, blood oxygen, an electrocardiogram, and blood pressure) of a user. For example, the intelligent wearable device may be a smartwatch, a smart band, smart glasses, or a smart helmet. Alternatively, the electronic device may be a mobile phone (mobile phone), a tablet computer (pad), a notebook computer, a smart home, a virtual reality (virtual reality, VR) electronic device, an augmented reality (augmented reality, AR) electronic device, or the like. Alternatively, the electronic device may be a handheld device that has a wireless communication function, a computing device or another processing device connected to a wireless modem, a vehicle-mounted device, an electronic device in a 5G network, an electronic device in a future evolved public land mobile network (public land mobile network, PLMN), or the like. This is not limited in embodiments of this application.

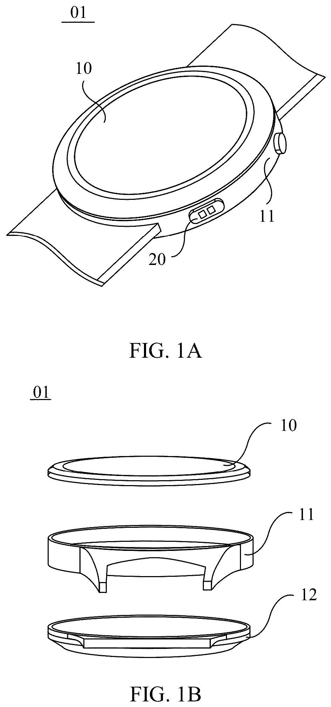

For ease of description, the following uses an example in which an electronic device 01 shown in FIG. 1A is a smartwatch that can perform health monitoring. For example, the electronic device 01 may include a display (display) 10, a middle frame 11, and a rear cover (rear cover) 12 shown in FIG. 1B. Still as shown in FIG. 1B, the display 10 may be disposed in the middle frame 11, a display surface of the display 10 is located on a side away from the rear cover 12, and the middle frame 11 may be disposed around the display 10. The display 10 may be a liquid crystal display (liquid crystal display, LCD), an organic light emitting diode (organic light emitting diode, OLED) display, or a micro (micro or mini) light emitting diode (light emitting diode, LED) display.

A shape of the display 10 is not limited in this application. For example, the display surface of the display 10 may be a circle or a rectangle. Contour shapes of the middle frame 11 and the rear cover 12 may match a contour shape of the display 10. The following uses an example in which the contour shapes of the display 10, the middle frame 11, and the rear cover 12 are circles for description.

On this basis, the electronic device 01 may further include components such as a circuit board, a battery, a processor, a sensor, a microphone, and a speaker. In some embodiments of this application, components such as the circuit board, the battery, the processor, the sensor, the microphone, and the speaker may be disposed between the display 10 and the rear cover 12. The middle frame 11 may support the entire electronic device 01. In some embodiments of this application, the rear cover 12 covers an upper edge and a lower edge of the middle frame 11 to form a casing or housing (housing) of the electronic device 01. Alternatively, the middle frame 11 and the rear cover 12 may be connected to form an integrally formed part, to form the casing or housing of the electronic device 01. It should be understood that the "casing or housing" may be used to represent a part or all of the rear cover 12 or the middle frame 11, or may be used to represent a part or all of any combination of the rear cover 12 and the middle frame 11.

For example, the processor may include one or more processing units. For example, the processor may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, and/or a neural-network processing unit (neural-network processing unit, NPU). Different processing units may be independent components, or may be integrated into one or more processors.

In addition, to monitor a health sign parameter, for example, data such as a heart rate, blood oxygen, an electrocardiogram, and blood pressure, the electronic device 01 may further include a monitoring apparatus 20 shown in FIG. 1A. The monitoring apparatus 20 may be connected to the middle frame 11. For example, an outer surface of the monitoring apparatus 20 may protrude from an outer surface of the middle frame 11. Alternatively, for another example, the outer surface of the monitoring apparatus 20 may be flush with the outer surface of the middle frame 11. This is not limited in this application.

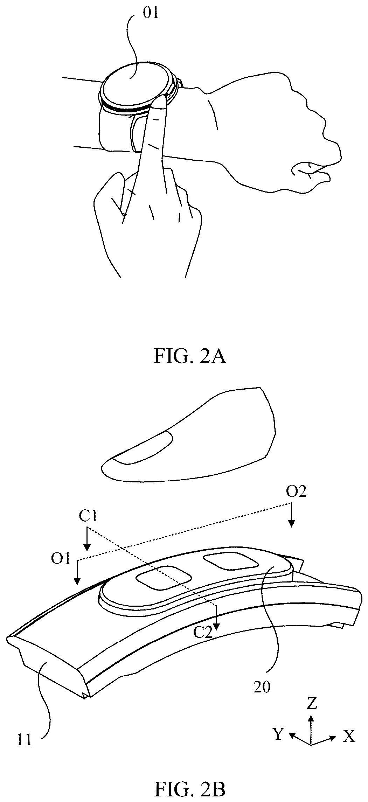

Based on this, when the electronic device 01 is a smartwatch, as shown in FIG. 2A, the user may wear the electronic device 01 on a left hand, and press the monitoring apparatus 20 with a finger of a right hand (as shown in FIG. 2B). Alternatively, the electronic device 01 may be worn on the right hand, and the monitoring apparatus 20 may be pressed with a finger of the left hand. In this case, as shown in FIG. 2B, in some embodiments of this application, when the user presses the monitoring apparatus 20 with the finger, the monitoring apparatus 20 may collect a heart rate, blood oxygen, and electrocardiogram data of the user. Alternatively, in some other embodiments, the monitoring apparatus 20 may further calculate a pressure value based on the heart rate, the blood oxygen, the electrocardiogram, and the like. Alternatively, the monitoring apparatus 20 may further collect a pressure value of pressing the monitoring apparatus 20 by the user, so that the electronic device 01 may calculate a blood pressure value of the user based on the heart rate, the blood oxygen, the electrocardiogram data, and the pressure value. For ease of detailed description of a structure of the monitoring apparatus 20, a coordinate system XYZ of the monitoring apparatus 20 is established in FIG. 2B. An X direction may be a length direction of the monitoring apparatus 20, a Y direction is a width direction of the monitoring apparatus 20, and a Z direction is a height direction of the monitoring apparatus 20.

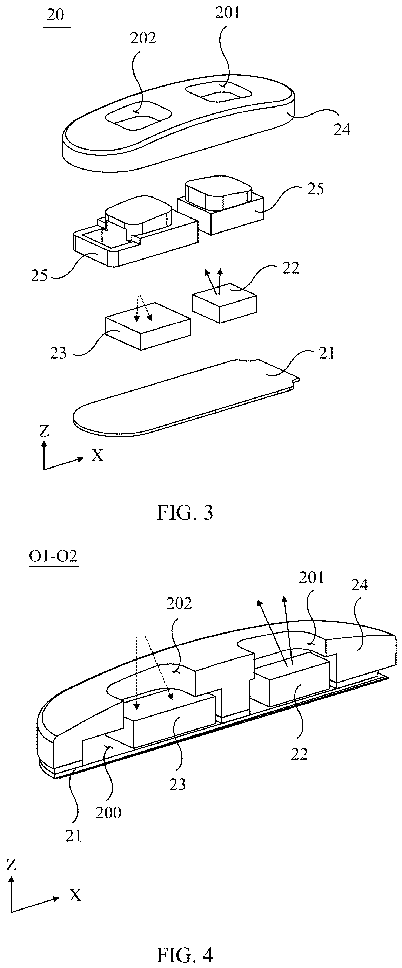

The following describes a structure of the monitoring apparatus 20 by using an example. In some embodiments of this application, as shown in FIG. 3, the monitoring apparatus 20 may include a first circuit board 21, a light emitting device 22, an optical-to-electrical converter 23, a metal cover plate 24, and a light transmission structure 25.

For example, the first circuit board 21 may include a flexible printed circuit (flexible printed circuit, FPC). Alternatively, for another example, the first circuit board 21 may include a PCB. The light emitting device 22 may be an LED, an OLED, or a quantum dot light emitting diode (quantum dot light emitting diode, QLED), and the user sends light for monitoring, for example, at least one of visible light and infrared light. The optical-to-electrical converter 23 may be a photodiode (photodiode, PD) or a phototransistor. The optical-to-electrical converter 23 can receive reflected light from the skin of the user and perform photoelectric conversion.

The light emitting device 22 and the optical-to-electrical converter 23 may be disposed on the first circuit board 21, and are electrically connected to the first circuit board 21. A circuit structure used to control the light emitting device 22 and the optical-to-electrical converter 23 may be disposed on the first circuit board 21. As shown in FIG. 4 (a sectional view obtained by performing sectioning along a dashed line O1-O2 in FIG. 2B, where the dashed line O1-O2 is parallel to the X direction), the light emitting device 22 and the optical-to-electrical converter 23 may be located on a same side of the first circuit board 21. In this way, the light emitting device 22 and the optical-to-electrical converter 23 may form a photo plethysmograph (photo plethysmograph, PPG) module.

A specific working principle of the PPG module may be as follows: Blood circulation is driven by contraction and relaxation of a beating heart. When the heart contracts, blood flows to the body, a to-be-monitored part is congested, and a blood volume increases. When the heart relaxes, blood in the to-be-monitored part flows back to the heart, and the blood volume decreases. In this way, a periodic signal is generated. The periodic signal may include a systolic period peak and a diastolic period peak. A heart rate (heart rate, HR) may be determined based on the periodic signal. In addition, blood oxygen content in blood is related to hemoglobin content.

Based on this, the skin of the human body transmits and reflects visible light and infrared light. After light emitted by the light emitting device 22 is irradiated to the skin, a flow state of blood in the skin (for example, a change of blood flow caused by a heartbeat) and hemoglobin content in the blood affect an amount of absorbed light, and further affect a light reflection state of the skin. The optical-to-electrical converter 23 collects light reflected by the skin, and can detect a rule of light intensity that is related to the blood volume and that changes with time of the human body, that is, a photoplethysmogram. A feature of blood flow and hemoglobin content may be reflected by using the photoplethysmogram, so that parameters such as the heart rate and the blood oxygen may be obtained.

On this basis, as shown in FIG. 4, the metal cover plate 24 of the monitoring apparatus 20 covers the first circuit board 21. In addition, an accommodation cavity 200 may be formed between the metal cover plate 24 and the first circuit board 21, and the light emitting device 22 and the optical-to-electrical converter 23 may be disposed in the accommodation cavity 200. A first light through hole 201 and a second light through hole 202 that penetrate the metal cover plate 24 are disposed on the metal cover plate 24. The first light through hole 201 exposes a light exit surface of the light emitting device 22, and the second light through hole 202 exposes a light receiving surface of the optical-to-electrical converter 23.

The light exit surface of the light emitting device 22 is a surface that is of the light emitting device 22 and that is used to emit light, and the light receiving surface of the optical-to-electrical converter 23 is a surface that is of the optical-to-electrical converter 23 and that is used to receive the light reflected by the skin. When a limb of the user, for example, a finger, touches a surface that is of the metal cover plate 24 and that is away from the first circuit board 21 (that is, an outer surface of the metal cover plate 24), because the first light through hole 201 on the metal cover plate 24 in FIG. 4 exposes the light exit surface of the light emitting device 22, light emitted by the light emitting device 22 can pass through the first light through hole 201 and be irradiated to the finger of the user. In addition, because the second light through hole 202 on the metal cover plate 24 exposes the light receiving surface of the optical-to-electrical converter 23, light reflected by the finger of the user may pass through the second light through hole 202 and be incident to the light receiving surface of the optical-to-electrical converter 23, so that the emitted light is collected, and PPG data can be obtained.

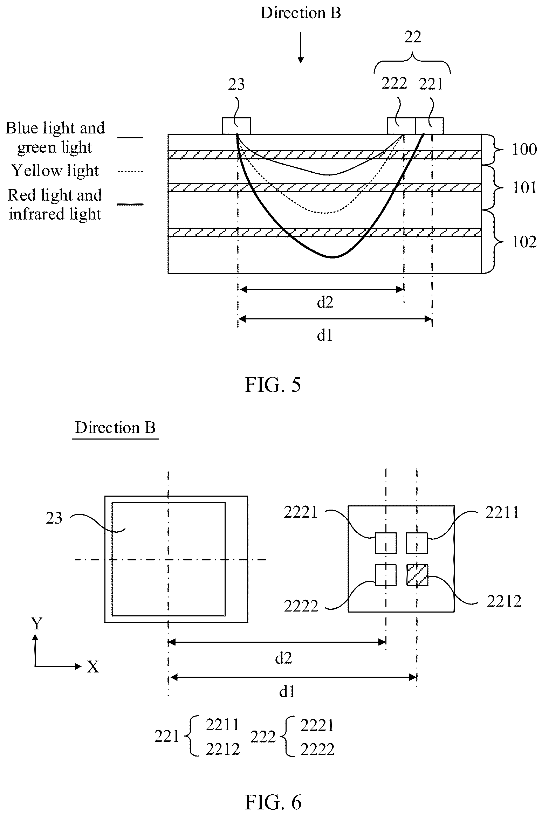

In addition, the skin of the human body may sequentially include an epidermis layer 100, a dermis layer 101, and subcutaneous tissue 102 shown in FIG. 5 from outside to inside. Based on this, in some embodiments of this application, the light emitting device 22 may emit visible light of different wavelengths, for example, at least one of blue light, green light, yellow light, and red light. In addition, the light emitting device 22 may further emit infrared light. Based on this, the light emitting device 22 may include a first light emitting component 221 and a second light emitting component 222 shown in FIG. 5. The first light emitting component 221 may emit at least one of the red light or the infrared light, and the second light emitting component 222 may emit at least one of the yellow light, the green light, and the blue light. Table 1 shows wavelengths of the visible light and the infrared light and monitoring targets.

**Table 1**

| Light emitting device | Wavelength | Monitoring target |
|---|---|---|
| Blue light | 400 nm to 505 nm | Capillary |
| Green light | 505 nm to 560 nm | |
| Yellow light | 580 nm to 600 nm | Small artery |
| Red light | 805 nm to 960 nm | Artery |
| Infrared light | 805 nm to 960 nm | |

Refer to Table 1. As shown in FIG. 5, the light emitted by the light emitting device 22 has different wavelengths, reaches different skin depths, and monitors different targets. For example, the wavelength of the blue light or the green light (the yellow light or the green light is represented by a fine solid line in FIG. 5) emitted by the second light emitting component 222 is short, and the blue light or the green light can reach the epidermis layer 100. Therefore, a blood volume of blood in the capillary (a shadow part of the epidermis layer 100 in FIG. 5) can be monitored. The yellow light (represented by a fine dashed line in FIG. 5) emitted by the second light emitting component 222 can reach the dermis layer 101, to monitor a blood volume of blood in the small artery (a shadow part of the dermis layer 101 in FIG. 5). The wavelengths of the red light emitted by the first light emitting component 221 and the infrared light emitted by the second light emitting component 222 (the red light and the infrared light are represented by using bold solid lines in FIG. 5) are long, and the red light and the infrared light can reach the subcutaneous tissue 102, to monitor a blood volume of blood in the artery (a shadow part of the subcutaneous tissue 102 in FIG. 5).

In this way, the processor in the electronic device 01 can obtain PPG signals that are of blood vessels at different layers in the skin and that are from the optical-to-electrical converter 23, and perform comprehensive processing on the multidimensional PPG signals, to obtain more accurate PPG data, for example, data of the blood oxygen and the heart rate. The red light is mainly absorbed by oxyhemoglobin in the blood, and the infrared light is mainly absorbed by hemoglobin and deoxygenated hemoglobin in the blood. Therefore, the processor may calculate blood oxygen saturation by measuring a proportion of hemoglobin combined with oxygen in the blood based on an absorption difference between the red light and the infrared light collected by the optical-to-electrical converter 23, to obtain blood oxygen data.

Still as shown in FIG. 5, in some embodiments of this application, there may be a first spacing d1 between the first light emitting component 221 and the optical-to-electrical converter 23, and the first spacing d1 may fall within a size range of 3.5 mm≤d1≤5.5 mm. The first spacing d1 may be a spacing between a size center position (or a light emitting center position) of the first light emitting component 221 and a size center position of the optical-to-electrical converter 23. When the first spacing d1 falls within the range of 3.5 mm≤d1≤5.5 mm, the light emitted by the first light emitting component 221 can be more fully and easily received by the optical-to-electrical converter 23 after being reflected by the skin of the user. Therefore, a signal collected by the optical-to-electrical converter 23 can be more accurate, and the PPG data obtained by the electronic device 01 is more accurate. For example, the first spacing d1 may be 3.5 mm, 4 mm, 4.5 mm, 5 mm, or 5.5 mm.

Based on this, in some embodiments of this application, because the blood oxygen data is related to the absorption difference between the red light and the infrared light, as shown in FIG. 6 (a top view obtained in a direction B in FIG. 5), the first light emitting component 221 may include a first light source 2211 configured to emit the red light and a second light source 2212 configured to emit the infrared light. The first light source 2211 and the second light source 2212 each may be at least one of self-luminous devices such as an LED, an OLED, and a QLED. There is the first spacing d1 between the optical-to-electrical converter 23 and any one of the first light source 2211 and the second light source 2212. In the Y direction, positions of the first light source 2211 and the second light source 2212 and a spacing between the first light source 2211 and the second light source 2212 are not limited in this application.

In addition, as shown in FIG. 5, there is a second spacing d2 between the second light emitting component 222 and the optical-to-electrical converter 23. The second spacing d2 may fall within a size range of 3.0 mm≤d1≤4.5 mm. Similarly, the second spacing d2 may be a spacing between a size center position (or a light emitting center position) of the second light emitting component 222 and the size center position of the optical-to-electrical converter 23. When the second spacing d2 falls within the range of 3.0 mm≤d1≤4.5 mm, the PPG data obtained by the electronic device 01 may be more accurate. For example, the second spacing d2 may be 3 mm, 3.5 mm, 4 mm, or 4.5 mm.

Based on this, it can be learned from Table 1 that both the blue light and the green light can reach the epidermis layer 100 to monitor the PPG signal in the capillary. Therefore, the second light emitting component 222 may include a third light source 2221 and a fourth light source 2222 shown in FIG. 6. The third light source 2221 may be configured to emit the blue light or the green light, and the fourth light source 2222 is configured to emit the yellow light. Similarly, the third light source 2221 and the fourth light source 2222 each may be at least one of self-luminous devices such as an LED, an OLED, or a QLED. There is the second spacing d2 between the optical-to-electrical converter 23 and any one of the third light source 2221 and the fourth light source 2222. In the Y direction, positions of the third light source 2221 and the fourth light source 2222 and a spacing between the third light source 2221 and the fourth light source 2222 are not limited in this application.

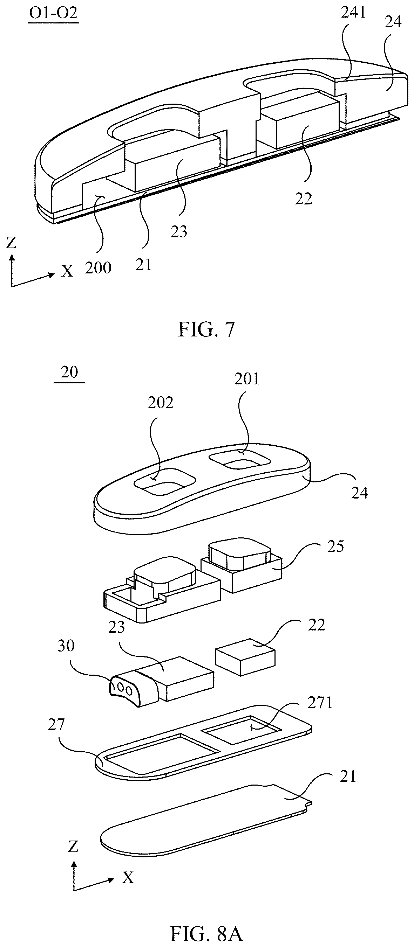

In addition, as shown in FIG. 7 (a sectional view obtained by performing sectioning along a dashed line O1-O2 in FIG. 2B), the metal cover plate 24 is electrically connected to the first circuit board 21, so that an electrical signal can be transmitted between the metal cover plate 24 and the first circuit board 21. Therefore, the metal cover plate 24 may be used as an electrocardiogram (electrocardiogram, ECG) electrode. Based on this, each beat of the heart causes a tiny electric wave on a skin surface. When the limb of the user, for example, the finger, touches the outer surface of the metal cover plate 24, the electric wave may be collected by the metal cover plate 24, and amplified by using the processor in the electronic device 01, to obtain an electrocardiogram. The electrocardiogram may be used to learn a health state of the heart of the user, for example, determine whether a heart activity is normal, slow, fast, or irregular.

The foregoing is an example for description by using an example in which the ECG data is monitored and the user directly contacts the outer surface of the metal cover plate 24. In some other embodiments of this application, the monitoring apparatus 20 may further include a metal coating 241 shown in FIG. 7. The metal coating 241 may cover the outer surface of the metal cover plate 24, that is, a surface that is of the metal cover plate 24 and that is away from the first circuit board 21. In this way, a material of the metal coating 241 may be used for appearance decoration. In addition, the metal cover plate 24 and the metal coating 241 may have a same conductivity or different conductivities. In this case, the ECG electrode may include the metal cover plate 24 and the metal coating 241. For ease of description, the metal coating 241 is not shown in some accompanying drawings in the following embodiments.

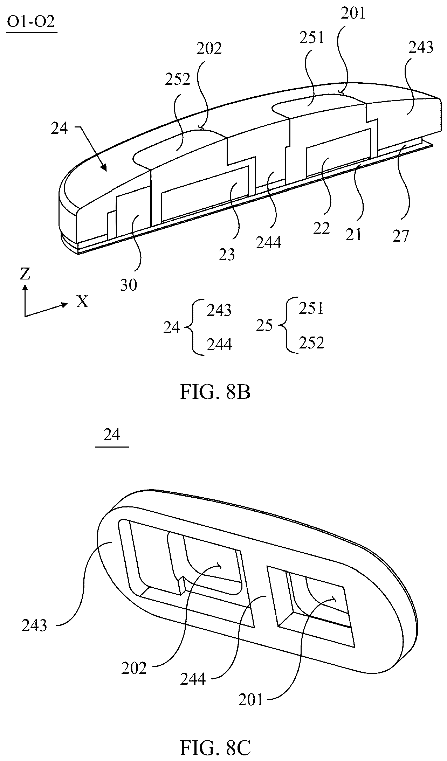

To electrically connect the metal cover plate 24 to the circuit board 21, in some embodiments of this application, as shown in FIG. 8A, the monitoring apparatus 20 may further include conductive silicone gel 30. The conductive silicone gel 30 may be disposed between the metal cover plate 24 and the first circuit board 21. As shown in FIG. 8B (a sectional view obtained by performing sectioning along a dashed line O1-O2 in FIG. 2B), a first end (for example, an upper end in a Z direction) of the conductive silicone gel 30 is electrically connected to the metal cover plate 24, and a second end (for example, a lower end in the Z direction) of the conductive silicone gel 30 is welded to the first circuit board 21. In this case, the two ends of the conductive silicone gel 30 may be separately welded to pads (pads) of the metal cover plate 24 and the first circuit board 21 by using a welding process, so that the metal cover plate 24 may be electrically connected to the first circuit board 21 through the conductive silicone gel 30, to implement signal interconnection between the conductive silicone gel 30 and the first circuit board 21.

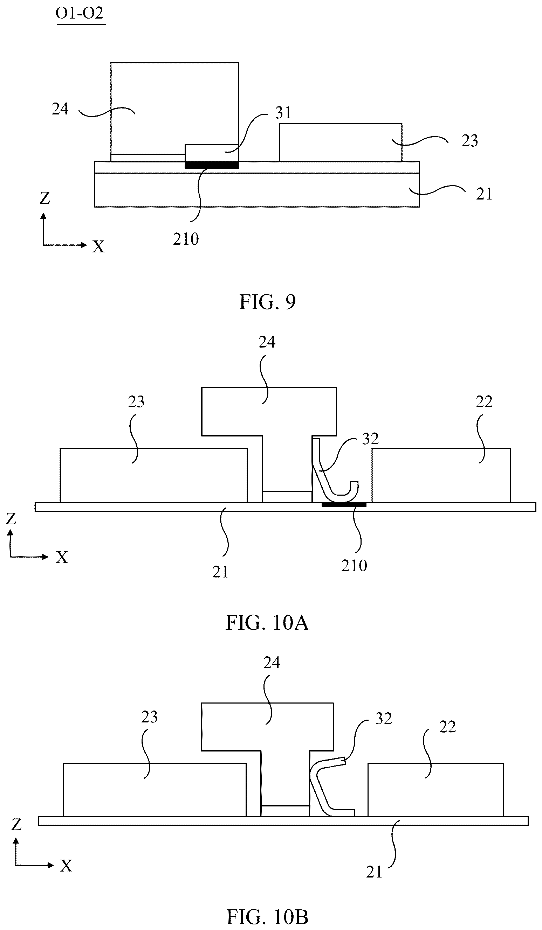

Alternatively, in some other embodiments of this application, as shown in FIG. 9, the monitoring apparatus 20 may further include conductive adhesive 31, disposed between the metal cover plate 24 and the first circuit board 21. The conductive adhesive 31 is bonded to the metal cover plate 24 and a copper exposure part 210 of the first circuit board 21, and the conductive adhesive 31 may be electrically connected to the metal cover plate 24 and the first circuit board 21. In this case, a part that is of the first circuit board 21 and that is electrically connected to the conductive adhesive 31 may not need to be provided with a pad (pad). The conductive adhesive 31 is bonded to the copper exposure part 210 of the first circuit board 21. A side that is of the conductive adhesive 31 and that is away from the copper exposure part 210 may be bonded to the metal cover plate 24, so that the metal cover plate 24 may implement signal interconnection with the first circuit board 21 through the conductive adhesive 31. For example, the copper exposure part 210 of the first circuit board 21 may be a part of a metal copper layer exposed in the first circuit board 21.

Alternatively, in some other embodiments of this application, the monitoring apparatus 20 further includes a metal dome 32 shown in FIG. 10A or FIG. 10B. The metal dome 32 may be disposed in the accommodation cavity 200 (as shown in FIG. 7). For example, as shown in FIG. 10A, a first end (that is, an end facing the metal cover plate 24) of the metal dome 32 is welded to the metal cover plate 24, and a second end (that is, an end facing the first circuit board 21) of the metal dome 32 abuts against the copper exposure part 210 of the first circuit board 21. In a process in which the second end of the metal dome 32 abuts against the copper exposure part 210 of the first circuit board 21, the metal dome 32 may elastically deform, and the deformed metal dome 32 may be in closer contact with the copper exposure part 210 of the first circuit board 21. Alternatively, as shown in FIG. 10B, the second end (that is, an end facing the first circuit board 21) of the metal dome 32 is welded to the first circuit board 21, and the first end (that is, an end facing the metal cover plate 24) of the metal dome 32 abuts against the metal cover plate 24. Similarly, in a process in which the first end of the metal dome 32 abuts against the metal cover plate 24, the metal dome 32 may elastically deform, and the deformed metal dome 32 may be in closer contact with the metal cover plate 24. In this way, the metal cover plate 24 may implement signal interconnection with the first circuit board 21 through the metal dome 32.

The foregoing is an example of an electrical connection manner between the metal cover plate 24 and the first circuit board 21, and does not constitute a limitation on the electrical connection manner. In addition, a position at which the metal cover plate 24 is electrically connected to the first circuit board 21 is not limited in this application.

It can be learned from the foregoing that PPG data such as the blood oxygen and the heart rate of the user may be obtained by using the PPG module (including the light emitting device 22 and the optical-to-electrical converter 23), and the ECG data may be obtained by using the ECG electrode, for example, the metal cover plate 24, or the metal cover plate 24 and the metal coating 241. Based on this, for example, pulse transit time (pulse transit time, PTT) may be obtained based on the foregoing PPG data and ECG data. A pulse wave transit speed may be obtained based on the PPT and parameters such as a height and a weight of the user, and a systolic blood pressure and a diastolic blood pressure of the user may be obtained based on the pulse wave transit speed, to implement non-invasive continuous blood pressure measurement. In in comparison with a cufftype blood pressure monitor, in a process of monitoring blood pressure by using the monitoring apparatus 20 provided in this application, the user does not need to wear the monitoring apparatus 20 on an arm, and only needs to touch the metal cover plate 24 with the finger. This can improve comfort and convenience of blood pressure monitoring.

It can be learned from the foregoing that the monitoring apparatus 20 provided in this embodiment of this application integrates the PPG module (the light emitting device 22 and the optical-to-electrical converter 23) and the ECG electrode. In addition, to connect the PPG module (the light emitting device 22 and the optical-to-electrical converter 23), the ECG electrode, and the first circuit board 21, the monitoring apparatus 20 may further include the light transmission structure 25 shown in FIG. 8B. The light transmission structure 25 may fill the first light through hole 201, the second light through hole 202, and the accommodation cavity 200 (as shown in FIG. 7).

Based on this, to improve comfort of pressing the monitoring apparatus 20 by the user, as shown in FIG. 8B, a surface that is of the light transmission structure 25 and that is away from the first circuit board 21 and a surface that is of the metal cover plate 24 and that is away from the first circuit board 21 may be spliced into an arc surface. In this way, the surface that is of the light transmission structure 25 and that is away from the first circuit board 21 may smoothly transit at a junction position of the surface that is of the metal cover plate 24 and that is away from the first circuit board 21, to improve pressing comfort of the user.

In addition, as shown in FIG. 8B, the light transmission structure 25 wraps the light emitting device 22 and the optical-to-electrical converter 23, and is connected to the light emitting device 22, the optical-to-electrical converter 23, the first circuit board 21, and the metal cover plate 24. A part of the light transmission structure 25 filling the first light through hole 201 and the accommodation cavity 200 may connect the light emitting device 22 to the first circuit board 21 and the metal cover plate 24, and the light transmission structure 25 has a light transmission feature, thereby reducing impact on light emitted from the light exit surface of the light emitting device 22. Similarly, a part of the light transmission structure 25 filling the second light through hole 202 and the accommodation cavity 200 may connect the optical-to-electrical converter 23 to the first circuit board 21 and the metal cover plate 24, and can reduce impact on light reflected from the skin to the light receiving surface of the optical-to-electrical converter 23. For ease of description, a part of the light transmission structure 25 that wraps the light emitting device 22 may be referred to as a first light transmission structure 251, and a part of the light transmission structure 25 that wraps the optical-to-electrical converter 23 may be referred to as a second light transmission structure 252.

For example, a light transmittance of the light transmission structure 25 may be greater than or equal to 80%. On this basis, because the light transmission structure 25 is light transmissive, to reduce optical crosstalk between the light emitting device 22 and the optical-to-electrical converter 23, for example, as shown in FIG. 8B, the metal cover plate 24 may include a metal cover plate body 243 and a light shielding blocking plate 244. The metal cover plate body 243 may cover the first circuit board 21, and the metal cover plate body 243 is electrically connected to the first circuit board 21. The electrical connection manner is the same as that described above, and is not described herein again. In addition, the first light through hole 201 and the second light through hole 202 that are spaced from each other may be disposed on the metal cover plate body 243, and a part of the metal cover plate body 243 is spaced between the first light through hole 201 and the second light through hole 202.

In addition, the light shielding blocking plate 244 is disposed in the accommodation cavity 200 (as shown in FIG. 7), and the light shielding blocking plate 244 may be connected to the metal cover plate body 243 and the first circuit board 21. As shown in FIG. 8C, the light shielding blocking plate 244 is located between the first light through hole 201 and the second light through hole 202, and may separate the light emitting device 22 and the optical-to-electrical converter 23 shown in FIG. 8B.

For example, materials of the metal cover plate body 243 and the material of the light shielding blocking plate 244 may be the same. For example, the light shielding blocking plate 244 may be prepared by using a metal material, and the light shielding blocking plate 244 may be connected to the metal cover plate body 243 to form an integrated structural part. For example, the integrated structural part may be formed by using a one-time metal casting process, a metal injection process, a 3D printing process, or a die casting process. In this way, because the light shielding blocking plate 244 is prepared by using a metal material, the light shielding blocking plate 244 has a light shielding performance. Therefore, disposing the light shielding blocking plate 244 between the light emitting device 22 and the optical-to-electrical converter 23 can reduce or even avoid direct incidence of light emitted by the light emitting device 22 on the optical-to-electrical converter 23. This can reduce optical crosstalk between the light emitting device 22 and the optical-to-electrical converter 23, and improve monitoring precision of the optical-to-electrical converter 23. Alternatively, the light shielding blocking plate 244 may be prepared by using a nonmetal light shielding material, and connected to the metal cover plate body 243 by bonding, clamping, threaded connection, or the like.

In addition, as shown in FIG. 8A, to reduce or avoid light leakage of light emitted by the light emitting device 22 from the bottom of the metal cover plate 24, that is, from a side facing the first circuit board 21, the monitoring apparatus 20 may further include a light shielding connection layer 27, disposed between the metal cover plate 24 and the first circuit board 21. As shown in FIG. 8B, the light shielding connection layer 27 may be connected to the light shielding blocking plate 244 and the first circuit board 21. For example, the light shielding connection layer 27 may be a light shielding adhesive layer or a light shielding adhesive tape. In this way, the bottom of the metal cover plate 24 may be bonded to the first circuit board 21 through the light shielding connection layer 27, to reduce or avoid light leakage of the light emitted by the light emitting device 22 from the bottom of the metal cover plate 24, and improve light utilization. Based on this, as shown in FIG. 8A, to dispose the light emitting device 22, the optical-to-electrical converter 23, and a component configured to electrically connect the metal cover plate 24 to the first circuit board 21, for example, the conductive silicone gel 30, an avoidance hole 271 may be disposed on the light shielding connection layer 27.

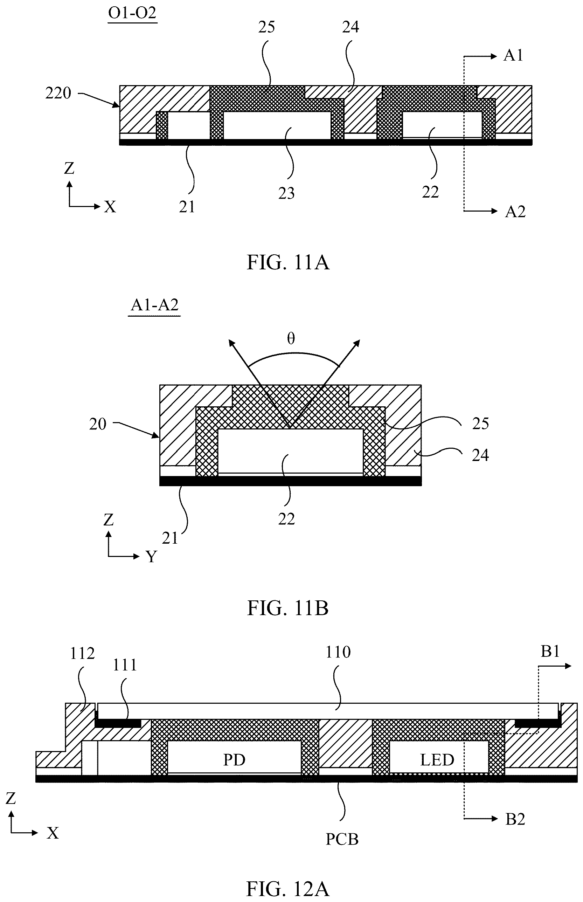

In conclusion, as shown in FIG. 11A, the light emitting device 22 and the optical-to-electrical converter 23 are covered in the metal cover plate 24, and the light transmission structure 25 may cover the light exit surface of the light emitting device 22 and the light receiving surface of the optical-to-electrical converter 23, to ensure normal operation of the light emitting device 22 and the optical-to-electrical converter 23. In addition, the light transmission structure 25 may further cover a side surface of the light emitting device 22 and a side surface of the optical-to-electrical converter 23 (parallel to a thickness direction, that is, the Z direction, of the monitoring apparatus 20). It can be learned from FIG. 11A (a sectional view obtained by performing sectioning along a dashed line O1-O2 in FIG. 2B) and FIG. 11B (a sectional view obtained by performing sectioning along a dashed line A1-A2 in FIG. 11A) that surfaces of the light emitting device 22 and the optical-to-electrical converter 23 other than surfaces facing the first circuit board 21 may be covered by the light transmission structure 25, so that the light transmission structure 25 can wrap the light emitting device 22 and the optical-to-electrical converter 23, and the light transmission structure 25 can be connected to the light emitting device 22, the optical-to-electrical converter 23, the first circuit board 21, and the metal cover plate 24 to form an integrated structural part. Based on this, when the light transmission structure 25 is made of a resin material, the light transmission structure 25 may be formed into an injection-molded integrated structural part 220 by using a one-time processing technology, for example, an injection molding technology. The injection-molded integrated structural part 220 may include the light emitting device 22, the optical-to-electrical converter 23, the first circuit board 21, the metal cover plate 24, and the light transmission structure 25 of the monitoring apparatus 20.

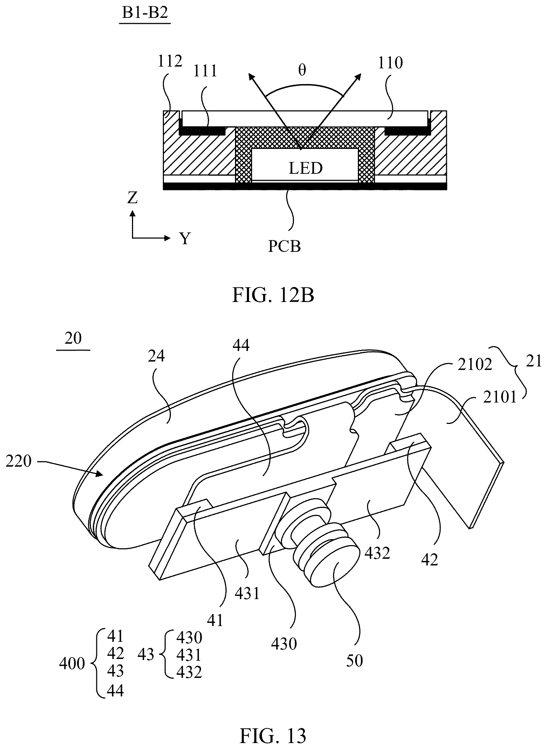

In the related technology shown in FIG. 12A, a transparent plate 110 is disposed above an LED and a PD disposed on a PCB. An ECG electrode (not shown in the figure) may be disposed on the transparent plate 110. The transparent plate may carry the ECG electrode. The transparent plate 110 is bonded to a bracket 112 through an adhesive layer 111. In this case, to ensure bonding firmness and waterproof performance between the transparent plate 110 and the bracket 112, a length of the adhesive layer 111 in the X direction needs to meet a specific requirement, and a length of the bracket 112 in the X direction also needs to meet a requirement for slotting (used to dispose the adhesive layer 111) and carrying the transparent plate 110. Therefore, a length of the entire device in the X direction is wide. Similarly, as shown in FIG. 12B (a sectional view obtained by performing sectioning along a dashed line B1-B2 in FIG. 12A), considering a width requirement of the adhesive layer 111 and the bracket 112 for carrying the transparent plate 110, a width of the entire device in the Y direction is wide.

Based on this, in one aspect, in comparison with the solution in FIG. 12A, the bracket 112, the transparent plate 110, and the adhesive layer 111 do not need to be disposed in this embodiment of this application. When emergent angles θ (as shown in FIG. 11B) of the light emitting devices 22 are the same, as shown in FIG. 11A, the monitoring apparatus 20 provided in this embodiment of this application may connect the metal cover plate 24 used as the ECG electrode to the light emitting device 22 and the optical-to-electrical converter 23 through the light transmission structure 25 embedded in the metal cover plate 24. Therefore, in the length direction

(that is, the X direction) of the monitoring apparatus, a length requirement of the adhesive layer and the bracket for carrying the transparent plate does not need to be considered. Similarly, in comparison with the solution in FIG. 12B, as shown in FIG. 11B, in the monitoring apparatus 20 provided in this embodiment of this application, in the width direction (that is, the Y direction) of the monitoring apparatus, the length requirement of the adhesive layer and the bracket for carrying the transparent plate does not need to be considered. Therefore, the length and the width of the monitoring apparatus 20 can be reduced. In addition, in this embodiment of this application, the light transmission structure 25 embedded in the metal cover plate 24 may cover surfaces that are of the light emitting device 22 and the optical-to-electrical converter 23 and that are away from the first circuit board 21. Therefore, the transparent plate 110 in FIG. 12A does not need to be disposed, so that a size in a height direction (that is, the Z direction) of the monitoring apparatus can be reduced. In this way, three-dimensional sizes of the monitoring apparatus 20 are all reduced, so that a purpose of miniaturization design of the monitoring apparatus 20 can be achieved. In this case, when the monitoring apparatus 20 with the miniaturization design is used in the electronic device 01, a size of the electronic device 01 can be reduced, so that a problem of large size of a device having a health sign monitoring function can be alleviated.

In addition, in the monitoring apparatus 20 that is formed by using the injection molding process and that is the integrated structural part, the light transmission structure 25 can not only connect the light emitting device 22 and the optical-to-electrical converter 23 to the metal cover plate 24 and the first circuit board 21, but also wrap surfaces and peripheries of the light emitting device 22 and the optical-to-electrical converter 23. Therefore, the light emitting device 22 and the optical-to-electrical converter 23 can be packaged. Impact of external water vapor and impurities on performance of the light emitting device 22 and the optical-to-electrical converter 23 can be reduced. This improves waterproof and dustproof reliability of the entire monitoring apparatus 20.

It can be learned from the foregoing that the monitoring apparatus 20 shown in FIG. 11A provided in this embodiment of this application has a PPG module (including the light emitting device 22 and the optical-to-electrical converter 23) and the ECG electrode (including the metal cover plate 24). The PPG module, the ECG electrode, and the first circuit board 21 may be connected through the light transmission structure 25 to form the injection-molded integrated structural part 220. Based on this, it can be learned from the foregoing that, in some embodiments of this application, when the finger of the user presses the metal cover plate 24, the PPG module may obtain the PPG data of the user, for example, data such as the heart rate and the blood oxygen. The ECG electrode may obtain the electrocardiogram data of the user. Blood pressure of the user may be obtained based on the PPG data and the electrocardiogram data. Alternatively, in some other embodiments of this application, in a process of obtaining blood pressure data of the user, a pressure value of pressing the metal cover plate 24 by the user may be introduced, and the blood pressure of the user may be calculated based on the PPG data, the ECG data, and the pressure value, to further improve blood pressure monitoring precision. In this case, to obtain the pressure value of pressing the metal cover plate 24 by the user, the monitoring apparatus 20 may further include a pressure sensing module. The following describes a structure of the pressure sensing module by using an example.

In some embodiments of this application, as shown in FIG. 13, a pressure sensing module 400 of the monitoring apparatus 20 may include a first lever 41, a second lever 42, a deformation plate 43, and a pressure-sensitive element 44. The first lever 41 and the second lever 42 are disposed on a side that is of the first circuit board 21 and that is away from the metal cover plate 24. For example, the first circuit board 21 may include an FPC 2101 and a supportive plate 2102. The supportive plate 2102 is disposed on a side that is of the FPC 2101 and that is away from the metal cover plate 24. A side that is of the FPC 2101 and that faces the metal cover plate 24 may be connected to the injection-molded integrated structural part 220 through an adhesive layer, and a side that is of the FPC 2101 and that is away from the metal cover plate 24 may be connected to the supportive plate 2102 through an adhesive layer. Rigidity of the supportive plate 2102 is greater than rigidity of the FPC 2101, so that the FPC 2101 can be supported. Based on this, the first lever 41 and the second lever 42 may be disposed on a side that is of the supportive plate 2102 and that is away from the metal cover plate 24. When the user presses the metal cover plate 24, a force transmitted to the first circuit board 21 may be transmitted to the first lever 41 and the second lever 42 through the supportive plate 2102.

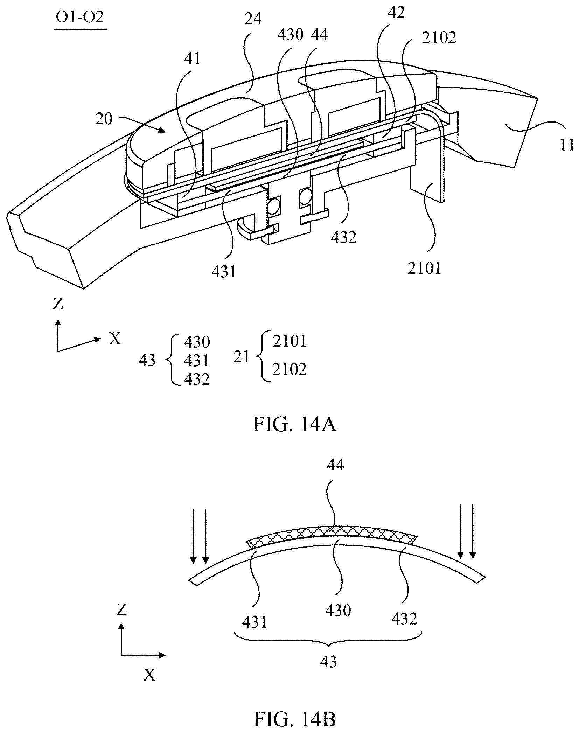

In addition, as shown in FIG. 13, the deformation plate 43 is disposed on a side that is of the first lever 41 and that is away from the first circuit board 21. The deformation plate 43 has a first cantilever end 431, a fastening end 430, and a second cantilever end 432. The fastening end 430 is located between the first cantilever end 431 and the second cantilever end 432. The deformation plate 43 is of a cantilever end structure. The fastening end 430 is configured to fasten the deformation plate 43. For example, the deformation plate 43 is fastened to the middle frame 11 shown in FIG. 14A. In this case, as shown in FIG. 13, a first end (for example, an upper end) of the first lever 41 may abut against or be connected to the first circuit board 21, and a second end (for example, a lower end) of the first lever 41 may abut against or be connected to the first cantilever end 431 of the deformation plate 43. A first end (for example, an upper end) of the second lever 42 may abut against or be connected to the first circuit board 21, and a second end (for example, a lower end) of the second lever 42 may abut against or be connected to the second cantilever end 432 of the deformation plate 43.

For example, as shown in FIG. 14A (a sectional view obtained by performing sectioning along a dashed line O1-O2 in FIG. 2B), the second end (for example, the lower end) of the first lever 41 may be connected to the first cantilever end 431 of the deformation plate 43 to form an integrated structural part, and the second end (for example, the lower end) of the second lever 42 may be connected to the second cantilever end 432 of the deformation plate 43 to form an integrated structural part. Based on this, in a process of assembling the monitoring apparatus 20, the first end (for example, the upper end) of the first lever 41 may be in contact with the first circuit board 21, for example, the supportive plate 2102 in the first circuit board 21, and the first end (for example, the upper end) of the second lever 42 may be in contact with the first circuit board 21, for example, the supportive plate 2102 in the first circuit board 21.

Alternatively, the first end (for example, the upper end) of the first lever 41 may be connected to the first circuit board 21, for example, the supportive plate 2102 in the first circuit board 21, to form an integrated structural part, and the first end (for example, the upper end) of the second lever 42 may be connected to the first circuit board 21, for example, the supportive plate 2102 in the first circuit board 21, to form an integrated structural part. Based on this, in a process of assembling the monitoring apparatus 20, the second end (for example, the lower end) of the first lever 41 may be in contact with the first cantilever end 431 of the deformation plate 43, and the second end (for example, the lower end) of the second lever 42 may be in contact with the second cantilever end 432 of the deformation plate 43.

In addition, as shown in FIG. 14A, the pressure-sensitive element 44 covers at least a part of the first cantilever end 431 of the deformation plate 43, and the pressure-sensitive element 44 further covers at least a part of the second cantilever end 432. In addition, the pressure-sensitive element 44 is electrically connected to the first circuit board 21, for example, the FPC 2101 in the first circuit board 21. In this way, in a process in which the finger of the user presses down the metal cover plate 24 in the Z direction, an external force applied by the user may be transmitted to the first circuit board 21 through the metal cover plate 24, and then transmitted to the first lever 41 and the second lever 42 that are located below the first circuit board 21 through the first circuit board 21. Because the first lever 41 and the second lever 42 respectively abut against or are connected to the first cantilever end 431 and the second cantilever end 432 of the deformation plate 43, and the fastening end 430 that is of the deformation plate 43 and that is located between the first cantilever end 431 and the second cantilever end 432 fastens the deformation plate 43 to a component, for example, the middle frame 11, the first cantilever end 431 and the second cantilever end 432 of the deformation plate 43 shown in FIG. 14B may deform downward in the Z direction under pressing action of the first lever 41 and the second lever 42. In addition, because the pressure-sensitive element 44 covers at least the part of the first cantilever end 431 and at least the part of the second cantilever end 432, the pressure-sensitive element 44 may convert deformation of the first cantilever end 431 and the second cantilever end 432 into an electrical signal by using a piezoelectric sensing principle, and transmit the electrical signal to the first circuit board 21 (shown in FIG. 14A) electrically connected to the pressure-sensitive element 44, to monitor a pressing force of the user.

As can be learned from the foregoing, the fastening end 430 of the deformation plate 43 is configured to fasten the deformation plate 43. The following describes a fastening manner of the fastening end 430 by using an example. For example, when the monitoring apparatus 20 is disposed on the middle frame 11, the monitoring apparatus 20 may be connected to the middle frame 11. The injection-molded integrated structural part 220 (including the light emitting device 22, the optical-to-electrical converter 23, the first circuit board 21, the metal cover plate 24, and the light transmission structure 25) and the deformation plate 43 may be disposed on an outer side of the middle frame 11. The fastening end 430 of the deformation plate 43 may be connected to an inner side of the middle frame 11.

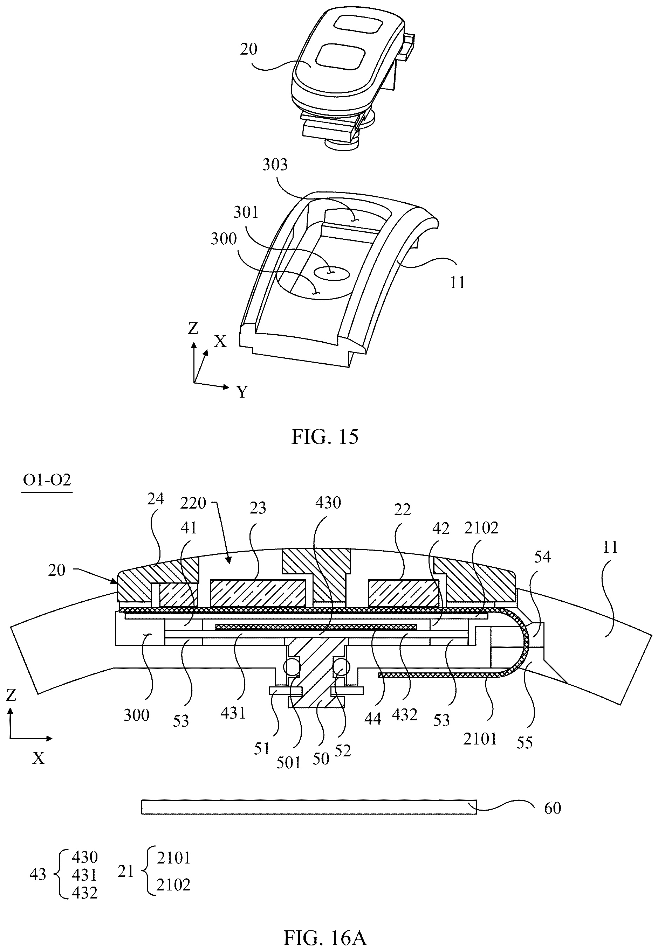

Based on this, in some embodiments of this application, as shown in FIG. 15, a mounting groove 300 is disposed on the outer surface of the middle frame 11, and a first mounting hole 301 that penetrates the middle frame 11 is disposed at a bottom of the mounting groove 300. On this basis, as shown in FIG. 16A (a sectional view obtained by performing sectioning along a dashed line O1-O2 in FIG. 2B), the injection-molded integrated structural part 220 (including the light emitting device 22, the optical-to-electrical converter 23, the first circuit board 21, the metal cover plate 24, and the light transmission structure 25), the first lever 41, the second lever 42, the deformation plate 43, and the pressure-sensitive element 44 may be disposed in the mounting groove 300, and the first circuit board 21 is close to the bottom of the mounting groove 300 relative to the metal cover plate 24. The pressure-sensitive element 44 may be disposed on a side that is of the deformation plate 43 and that faces the metal cover plate 24. Alternatively, in some other embodiments, the pressure-sensitive element 44 may be disposed on a side that is of the deformation plate 43 and that is away from the metal cover plate 24.

In addition, to fasten the fastening end 430 of the deformation plate 43, the monitoring apparatus further includes a first clamping ring 51 and a first sealing ring 52 shown in FIG. 16A. A mounting pillar 50 is located on a side that is of the deformation plate 43 and that is away from the metal cover plate 24. A first end (for example, an upper end) of the mounting pillar 50 is connected to the fastening end 430 of the deformation plate 43, and a second end (for example, a lower end) of the mounting pillar 50 is configured to fasten the monitoring apparatus. For example, the second end of the mounting pillar 50 penetrates the first mounting hole 301 (as shown in FIG. 15) and extends into the middle frame 11, the first clamping ring 51 is located in the middle frame 11, the first clamping ring 51 is clamped on the second end of the mounting pillar 50, and the first clamping ring 51 abuts against the middle frame 11. The mounting pillar 50 and the pressure-sensitive element 44 are respectively disposed on two opposite sides of the deformation plate, so that a disposing position of the pressure-sensitive element 44 does not affect a disposing position of the mounting pillar 50. In this way, in a process in which the user presses the monitoring apparatus 20, the first clamping ring 51 may limit movement of the mounting pillar 50 in the Z direction. In addition, when the first clamping ring 51 clamped to the second end of the mounting pillar 50 abuts against the middle frame 11, the mounting pillar 50 may be fastened to the middle frame 11 through the first clamping ring 51. The first end of the mounting pillar 50 is connected to the fastening end 430 of the deformation plate 43, so that the fastening end of the deformation plate 43 is fastened to the middle frame 11 through the mounting pillar 50.

On this basis, as shown in FIG. 16A, a first sealing groove 501 is disposed on a part that is of the mounting pillar 50 and that is located in the first mounting hole 301 (as shown in FIG. 15). The first sealing ring 52 may be located in the first sealing groove 501, and the first sealing ring 52 abuts against a hole wall of the first mounting hole 301 (as shown in FIG. 15) and a groove wall of the first sealing groove 501. Therefore, the first sealing ring 52 may be used to seal a gap between the first mounting hole 301 and the mounting pillar 50, to reduce a probability that external water vapor enters the middle frame 11 through the mounting groove 300 and the first mounting hole 301, and improve reliability of the product.

In addition, a third mounting hole 303 that penetrates the middle frame is disposed on the bottom of the mounting groove 300 shown in FIG. 15, and a part of the FPC 2101 (shown in FIG. 16A) may be disposed in the middle frame 11 after penetrating the third mounting hole 303. Based on this, the electronic device 01 may further include a cap 54, a second bonding layer 55, and a second circuit board 60 shown in FIG. 16A. The cap 54 may be disposed in the third mounting hole 303 (as shown in FIG. 15), and the cap 54 may block the third mounting hole 303. In addition, the second bonding layer 55 may be disposed in the third mounting hole 303, and is located on a side that is of the cap 54 and that is away from the mounting groove 300 (as shown in FIG. 16A). The second bonding layer 55 may be bonded to the cap 54 and the middle frame 11. The second circuit board 60 is disposed in the middle frame 11, and a part of the FPC 2101 penetrates the cap 54 and the second bonding layer 55 to extend into the middle frame 11, and is electrically connected to the second circuit board 60.

The second circuit board 60 may be referred to as a main board, and a processor may be disposed on the main board. After a part that is of the FPC 2101 and that is located in the middle frame 11 is electrically connected to the main board, the processor may transmit a signal to the metal cover plate 24, the light emitting device 22, the optical-to-electrical converter 23, and the pressure-sensitive element 44 in the monitoring apparatus 20 through the FPC 2101. In addition, the cap 54 may block the third mounting hole 303 (as shown in FIG. 15), to implement waterproofing and dustproofing. The second bonding layer 55 may be glue, and is used to bond the cap 54 to the middle frame 11.

It can be learned from the foregoing that the deformation plate 43 shown in FIG. 16A may deform under the pressing force applied by the user. For example, the deformation plate 43 may be a metal plate, and a material of the deformation plate 43 includes at least one of alloy steel, for example, titanium alloy and aluminum alloy. In addition, after the user cancels the pressing force, the deformation plate 43 needs to return to an original position. Therefore, the deformation plate 43 needs to undergo elastic deformation. It can be learned from the foregoing that the monitoring apparatus provided in this embodiment of this application has a small size. Therefore, a size of the deformation plate 43 is correspondingly reduced. However, after the size of the deformation plate 43 is reduced, when an external force is applied to press the deformation plate 43, a deflection of the deformation plate 43 is reduced. As a result, plastic deformation is likely to occur when the external force is relatively large.

Based on this, to reduce a probability of plastic deformation of the deformation plate 43 under the pressing action of the user, the monitoring apparatus may further include an elastic layer 53 shown in FIG. 16A. The elastic layer 53 may be located in the mounting groove 300, and the elastic layer 53 may be disposed between the deformation plate 43 and a bottom of the mounting groove 300. For example, the elastic layer 53 may be located on a side that is of the first cantilever end 431 and the second cantilever end 432 of the deformation plate 43 and that is away from the metal cover plate 24. The elastic layer 53 may cover a part of the first cantilever end 431 and the second cantilever end 432, or may completely cover the first cantilever end 431 and the second cantilever end 432.

In some embodiments of this application, the elastic layer 53 may include silicone gel or rubber. A modulus of the silicone gel or the rubber may increase as compression deformation increases. In this way, when the user applies large pressing pressure, a modulus of the elastic layer 53 increases after the elastic layer 53 is pressed, to support the deformation plate 43, thereby implementing a limiting function, so that the first cantilever end 431 and the second cantilever end 432 of the deformation plate 43 are not easy to plastically deform. For example, when a spacing between the deformation plate 43 and the middle frame 11 is small, room temperature vulcanized silicone rubber (room temperature vulcanized silicone rubber, RTV) may be used to dispense glue between the deformation plate 43 and the middle frame 11, and then the injection-molded integrated structural part 220, the first lever 41, the second lever 42, and the like are placed in the mounting groove until the glue is cured to form the elastic layer 53. The formed elastic layer 53 may fill a gap between the deformation plate 43 and the middle frame 11.

The above is an example for description that the elastic layer 53 may be the silicone gel or rubber. In some other embodiments of this application, the elastic layer 53 may alternatively be a tape or foam adhesive with a soft material.

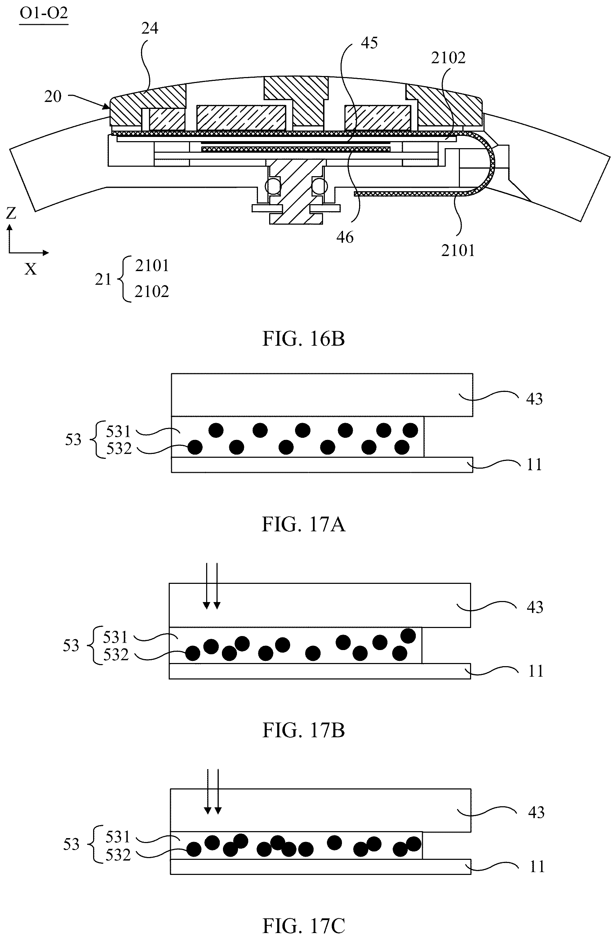

Alternatively, in some other embodiments of this application, as shown in FIG. 17A, the elastic layer 53 may further include a soft rubber layer 531 and a plurality of particles 532 doped in the soft rubber layer 531. Hardness of the particles 532 may be greater than hardness of the soft rubber layer 531. The soft rubber layer 531 includes silicone gel or rubber. The particles 532 may include metal particles, plastic particles, or rubber particles with high hardness. In this way, as shown in FIG. 17B, under the pressing force (in an arrow direction) of the user, the soft rubber layer 531 elastically deforms under pressure, so that a spacing between the particles 532 is reduced. Further, as shown in FIG. 17C, at least some of the particles 532 directly contact each other, so that rigidity of the entire elastic layer 53 is increased, and the deformation plate 43 can be supported, thereby achieving limiting effect. Therefore, the first cantilever end 431 and the second cantilever end 432 of the deformation plate 43 are not easy to plastically deform.

It can be learned from the foregoing that in a process in which the user presses the monitoring apparatus 20, the first cantilever end 431 and the second cantilever end 432 of the deformation plate 43 shown in FIG. 14B may deform downward in the Z direction. In this case, the pressure-sensitive element 44 covering at least a part of the first cantilever end 431 and at least a part of the second cantilever end 432 may convert deformation of the first cantilever end 431 and the second cantilever end 432 into an electrical signal by using a piezoelectric sensing principle, to monitor the pressing force of the user. The following describes an example of a structure of the pressure-sensitive element 44 that can implement the foregoing piezoelectric sensing.

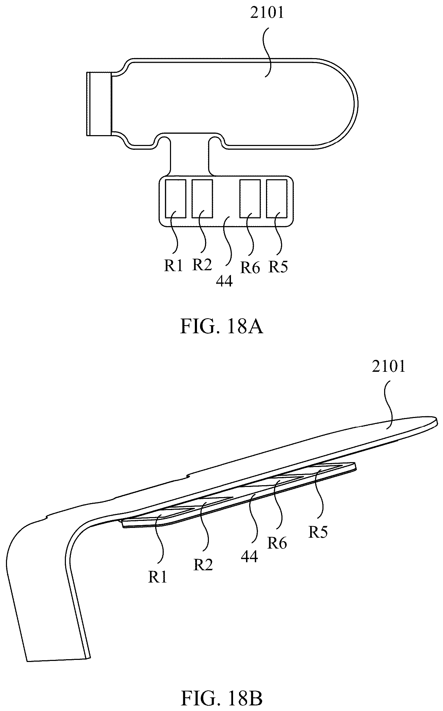

In some embodiments of this application, when the first circuit board 21 includes the FPC 2101 shown in FIG. 18A, the pressure-sensitive element 44 may be connected to the FPC 2101 to form an integrated structure. In a process of assembling the monitoring apparatus, as shown in FIG. 18B, the pressure-sensitive element 44 may be folded to a position on which the pressure-sensitive element 44 is stacked with the FPC 2101, so that the pressure-sensitive element 44 may be disposed on a side that is of the deformation plate 43 and that faces the metal cover plate 24 in FIG. 16A.

In addition, the pressure-sensitive element 44 may include a resistance strain gauge, and the resistance strain gauge may include a metal grid structure, a polymer ink structure, or the like. A pressure sensing monitoring principle of the resistance strain gauge may be that a resistance value of the metal grid structure, the polymer ink structure, or the like in the resistance strain gauge is related to not only a property of a material, but also a size such as a length and a cross-sectional area of the metal grid structure or the polymer ink structure. The resistance strain gauge is attached to the deformation plate 43. When the deformation plate 43 deforms under a force, the size such as the length and the cross-sectional area of the metal grid structure or the polymer ink structure also changes with the deformation plate 43, so that the resistance value of the resistance strain gauge changes.

Based on this, as shown in FIG. 18A or FIG. 18B, the resistance strain gauge may include a first resistor R1, a second resistor R2, a fourth resistor R4, and a fifth resistor R5. The first resistor R1, the second resistor R2, the fourth resistor R4, and the fifth resistor R5 are all variable resistors. A part that is of the pressure-sensitive element 44 and that covers the first cantilever end 431 shown in FIG. 16A has the first resistor R1 and the second resistor R2, and a part that is of the pressure-sensitive element 44 and that covers the second cantilever end 432 shown in FIG. 16A has the fourth resistor R4 and the fifth resistor R5.

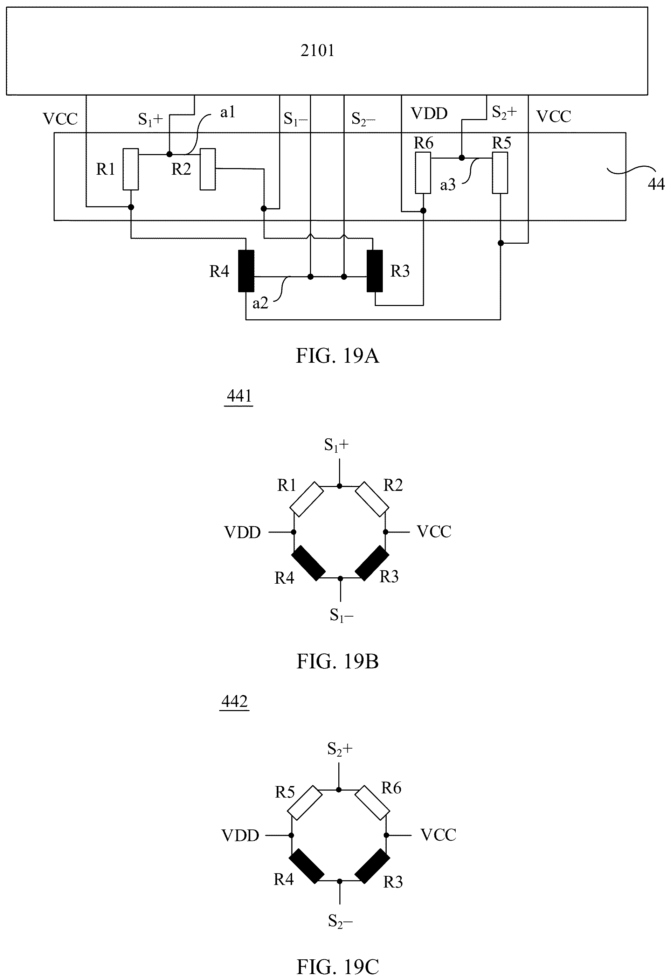

In this case, the monitoring apparatus 20 may further include a third resistor R3 and a fourth resistor R4 shown in FIG. 19A. The third resistor R3 and the fourth resistor R4 are resistors with fixed resistance values. For example, the third resistor R3 and the fourth resistor R4 may be disposed on a part that is of the FPC 2101 and that extends into the middle frame 11 in FIG. 16A. Alternatively, for another example, the third resistor R3 and the fourth resistor R4 may be disposed on a mainboard located in the middle frame 11, for example, the second circuit board 60 shown in FIG. 16A. A disposing position of the third resistor R3 and the fourth resistor R4 is not limited in this application, provided that the third resistor R3 and the fourth resistor R4 are not disposed on the pressure-sensitive element 44, to reduce a size of the pressure-sensitive element 44.

Based on this, as shown in FIG. 19A, the first resistor R1 and the second resistor R2 are connected in series to form a first circuit a1, and the third resistor R3 and the fourth resistor R4 are connected in series to form a second circuit a2. The first circuit a1 and the second circuit a2 may form a first full-bridge circuit 441 shown in FIG. 19B. The first full-bridge circuit 441 may be electrically connected to the FPC 2101 (shown in FIG. 19A) in the first circuit board through a first voltage end VDD, a second voltage end VCC, a first signal end S1 +, and a second signal end S1-. The first voltage end VDD and the second voltage end VCC are power supply ends of the first full-bridge circuit 441. The first voltage end VDD may supply a working voltage to the first full-bridge circuit 441, and the second voltage end VCC may be grounded.

In this way, as shown in FIG. 16A, when the user presses the metal cover plate 24 of the monitoring apparatus, the first cantilever end 431 of the deformation plate 43 deforms. In this case, resistance values of the first resistor R1 and the second resistor R2 in the pressure-sensitive element 44 that covers the first cantilever end 431 shown in FIG. 19B change, so that a voltage difference between the first signal end S1+ and the second signal end S1- changes. The voltage difference matches a pressure at which the user presses the metal cover plate 24 (shown in FIG. 16A), so that the pressing pressure of the user, for example, the first pressure value, may be monitored by obtaining the voltage difference.

Similarly, as shown in FIG. 19A, the fifth resistor R5 and the sixth resistor R6 are connected in series to form a third circuit a3, and the third resistor R3 and the fourth resistor R4 are connected in series to form the second circuit a2. The second circuit a2 and the third circuit a3 may form a second full-bridge circuit 442 shown in FIG. 19C. The second full-bridge circuit 442 may be electrically connected to the FPC 2101 (shown in FIG. 19A) in the first circuit board through the first voltage end VDD, the second voltage end VCC, a third signal end S2+, and a fourth signal end S2-. As shown above, the first voltage end VDD and the second voltage end VCC are power supply ends of the second full-bridge circuit 442. The first voltage end VDD may supply a working voltage to the second full-bridge circuit 442, and the second voltage end VCC may be grounded.

In this way, as shown in FIG. 16A, when the user presses the metal cover plate 24 of the monitoring apparatus 20, the second cantilever end 432 of the deformation plate 43 deforms. In this case, resistance values of the fifth resistor R5 and the sixth resistor R6 in the pressure-sensitive element 44 that covers the second cantilever end 432 shown in FIG. 19C change, so that a voltage difference between the third signal end S2+ and the fourth signal end S2- changes. The voltage difference matches a pressure at which the user presses the metal cover plate 24 (shown in FIG. 16A), so that the pressing pressure of the user, for example, the second pressure value, may be monitored by obtaining the voltage difference.

It can be learned from the foregoing that, when the user presses the metal cover plate 24 of the monitoring apparatus 20, the user pressing pressure detected by the first full-bridge circuit 441 shown in FIG. 19B, for example, the first pressure value, comes from a deformation amount of the first cantilever end 431 of the deformation plate 43 in FIG. 16A, and the user pressing pressure detected by the second full-bridge circuit 442 shown in FIG. 19C, for example, the second pressure value, comes from a deformation amount of the second cantilever end 432 of the deformation plate 43. The first cantilever end 431 and the second cantilever end 432 are respectively located on two sides of the fastening end 430. Therefore, the processor of the electronic device 01 may determine, by comparing a difference between the first pressure value and the second pressure value, whether a pressing position of the user is in a middle position of the metal cover plate 24, to improve monitoring precision. Therefore, the monitoring apparatus 20 shown in FIG. 16A may be referred to as a dual-channel monitoring structure.

For example, when the difference between the first pressure value and the second pressure value is large, the pressing position of the user is biased toward a side with a large pressure value. When the difference between the first pressure value and the second pressure value is small, the pressing position of the user is located at a middle position of the metal cover plate 24. In this way, the user can be reminded in time to change the pressing position, so that the pressing position is at the middle position of the metal cover plate 24, to improve monitoring precision.

On this basis, to enable the user to press a preset position, a plurality of protrusion structures may be formed on the outer surface of the metal cover plate 24. The user may clearly feel a concave-convex feeling of a touch surface in a pressing process. A position of the protrusion structure may match a preset pressing position, to guide the user to press the preset position, thereby improving monitoring precision.

In addition, in the dual-channel monitoring structure provided in this embodiment of this application, as shown in FIG. 19A, only four variable resistors, that is, the first resistor R1, the second resistor R2, the fifth resistor R5, and the sixth resistor R6, need to be disposed on the pressure-sensitive element 44. The first full-bridge circuit 441 (shown in FIG. 19B) including the first resistor R1, the second resistor R2, and a third resistor R3 and a fourth resistor R4 that are located outside the pressure-sensitive element 44 may share the third resistor R3 and the fourth resistor R4 with the second full-bridge circuit 442 including the fifth resistor R5, the sixth resistor R6, and the third resistor R3 and the fourth resistor R4 that are located outside the pressure-sensitive element 44. In this way, eight variable resistors used to form the foregoing two full-bridge circuits do not need to be directly disposed on the pressure-sensitive element 44, to reduce a size of the pressure-sensitive element 44, thereby further reducing a size of the entire monitoring apparatus 20 and even a size of the electronic device 01.

The foregoing is an example for description by using an example in which the monitoring apparatus 20 shown in FIG. 16A uses a resistive pressure sensing manner. In some other embodiments of this application, the monitoring apparatus 20 may further use a capacitive pressure sensing manner. In this case, as shown in FIG. 16B, the monitoring apparatus may further include a first metal electrode plate 45 disposed on a side that is of the supportive plate 2102 and that is away from the metal cover plate 24. The first metal electrode plate 45 may be electrically connected to the FPC 2101. In addition, the pressure-sensitive element may include a second metal electrode plate 46 in FIG. 16B, and a capacitor may be formed between the first metal electrode plate 45 and the second metal electrode plate 46. In this way, when the user presses the metal cover plate 24 of the monitoring apparatus 20, a spacing between the foregoing two metal electrodes changes, so that a capacitance value of the capacitor can be changed. The capacitance value matches the pressing pressure of the user, so that an objective of monitoring the pressing pressure of the user can be achieved. Alternatively, in some other embodiments, when the supportive plate 2102 includes a metal material, the supportive plate 2102 may be used as one electrode of the capacitor. In addition, the pressure-sensitive element 44 may be prepared by using a metal material, to be used as the other electrode of the capacitor, to implement the capacitive pressure sensing manner.

Alternatively, in some other embodiments of this application, the monitoring apparatus 20 may further use an inductive pressure sensing manner. In this case, the supportive plate 2102 shown in FIG. 16A may be prepared by using a metal material, and the pressure-sensitive element 44 may include a coil. In this case, a current may be supplied to the coil. When the user presses the metal cover plate 24 of the monitoring apparatus 20, a spacing between the supportive plate 2102 and the pressure-sensitive element 44 changes, so that a magnetic field generated by the coil may be changed, and impedance of the coil changes. The impedance may match a pressing force of the user, to monitor the pressing force of the user. Alternatively, in some other embodiments, the monitoring apparatus may further include a metal electrode plate disposed on a side that is of the supportive plate 2102 and that is away from the metal cover plate 24. The pressure-sensitive element 44 may include a coil, to implement the inductive pressure sensing manner.

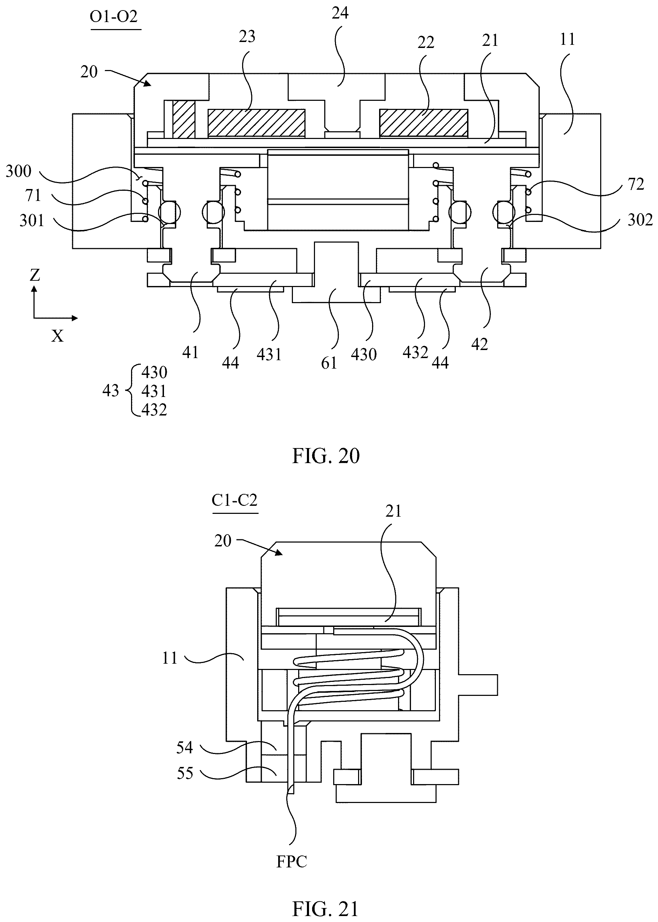

It can be learned from the foregoing that the monitoring apparatus 20 having a pressure sensing function shown in FIG. 16A is described by using an example in which the pressure-sensitive element 44 in the monitoring apparatus 20 is disposed outside the middle frame 11 and the monitoring apparatus is the dual-channel monitoring structure. In some other embodiments of this application, the pressure-sensitive element 44 of the monitoring apparatus 20 may be disposed inside the middle frame 11. Specifically, as shown in FIG. 20 (a sectional view obtained by performing sectioning along a dashed line O1-O2 in FIG. 2B), the monitoring apparatus 20 includes the first circuit board 21, the light emitting device 22, the optical-to-electrical converter 23, the metal cover plate 24, the first lever 41, the second lever 42, the deformation plate 43, the pressure-sensitive element 44, and the like. The first circuit board 21 may be a PCB. A disposition manner and a working process of the light emitting device 22, the optical-to-electrical converter 23, and the metal cover plate 24 are the same as those described above, and details are not described herein again.

In this embodiment of this application, the middle frame 11 is a ring structure having a hollow region, and may be a circular ring or a rectangular ring. The outside of the middle frame 11 or an outer side of the middle frame 11 refers to a side that is of the middle frame 11 and that is away from the hollow region. The inside of the middle frame 11 or an inner side of the middle frame 11 refers to a side that is of the middle frame 11 and that faces the hollow region.

In addition, as shown in FIG. 20, the deformation plate 43 is located inside the middle frame 11, and the deformation plate 43 is disposed on a side that is of the first lever 41 and the second lever 42 and that is away from the first circuit board 21. The deformation plate 43 has the first cantilever end 431, the second cantilever end 432, and the fastening end 430 located between the first cantilever end 431 and the second cantilever end 432. The first lever 41 penetrates the first mounting hole 301. The first lever 41 is disposed on a side that is of the first circuit board 21 and that is away from the metal cover plate 24. The first end (for example, the upper end) of the first lever 41 abuts against or is connected to the first circuit board 21, and the second end (for example, the lower end) of the first lever 41 extends out of the first mounting hole 301, is located inside the middle frame 11, and abuts against or is connected to the first cantilever end 431 of the deformation plate 43. In addition, the second lever 42 penetrates the second mounting hole 302. The second lever 42 is disposed on a side that is of the first circuit board 21 and that is away from the metal cover plate 24. The first end (for example, the upper end) of the second lever 42 abuts against or is connected to the first circuit board 21, and the second end (for example, the lower end) of the second lever 42 extends out of the second mounting hole 302, is located inside the middle frame 11, and abuts against or is connected to the second cantilever end 432. A manner in which the first lever 41 and the second lever 42 are fastened to the middle frame 11 through a clamping ring, and a manner in which the first lever 41 and the second lever 42 are sealed with the middle frame 11 through a sealing ring are the same as those described above, and details are not described herein again.

On this basis, to fasten the fastening end 430 of the deformation plate 43, the electronic device may further include a threaded connector 61 shown in FIG. 20. The threaded connector 61 may be a bolt or a screw. The threaded connector 61 may be located inside the middle frame 11, the threaded connector 61 penetrates the fastening end 430 of the deformation plate 43, and the threaded connector 61 is connected to the middle frame 11, to fasten the deformation plate 43 to the middle frame 11.

In addition, the pressure-sensitive element 44 is located inside the middle frame 11, the pressure-sensitive element 44 is disposed on a side that is of the deformation plate 43 and that is away from the metal cover plate 24, and the pressure-sensitive element 44 covers at least a part of the first cantilever end 431 and at least a part of the second cantilever end 432. The pressure-sensitive element 44 may be electrically connected to the first circuit board 21. In the pressure-sensitive element 44, a part covering the first cantilever end 431 and a part covering the second cantilever end 432 may be connected to form an integrated structure, or may not be connected and disposed separately. This is not limited in this application. In addition, a disposing manner and a working process of the pressure-sensitive element 44 are the same as those described above, and details are not described herein again. In this embodiment, because the pressure-sensitive element 44 is disposed in the middle frame 11, impact of external water vapor or impurities on the pressure-sensitive element 44 can be reduced, thereby improving pressure monitoring accuracy.

On this basis, to facilitate restoration of the deformation plate 43 to an initial position after the user cancels pressing force on the metal cover plate 24, the electronic device may further include a first spring 71 and a second spring 72 shown in FIG. 20. The first spring 71 is located in the mounting groove 300, a part of the first lever 41 penetrates the first spring 71, and two ends of the first spring 71 abut against the first lever 41 and a groove bottom of the mounting groove 300 respectively. In addition, the second spring 72 is located in the mounting groove 300, a part of the second lever 42 penetrates the second spring 72, and two ends of the second spring 72 abut against the second lever 42 and the groove bottom of the mounting groove 300, respectively. Based on this, to enable the first lever 41 to abut against the first spring 71, an end that is of the first lever 41 and that abuts against the first spring 71 may be disposed in a stepped shape. Similarly, to enable the second lever 42 to abut against the second spring 72, an end that is of the second lever 42 and that abuts against the second spring 72 may be disposed in a stepped shape. When the user does not press the monitoring apparatus 20, the first spring 71 and the second spring 72 may be in a compressed state. Therefore, compression amounts of the first spring 71 and the second spring 72 may be adjusted by adjusting a spacing between the stepped structure and the groove bottom of the mounting groove 300.

In addition, as shown in FIG. 21 (a sectional view obtained by performing sectioning along a dashed line C1-C2 in FIG. 2B), the electronic device may further include an FPC. One end of the FPC may be electrically connected to the first circuit board 21 located in the mounting groove 300, and the other end of the FPC may penetrate the cap 54 and the second bonding layer 55 to extend into the middle frame 11, to be electrically connected to a mainboard in the middle frame 11.

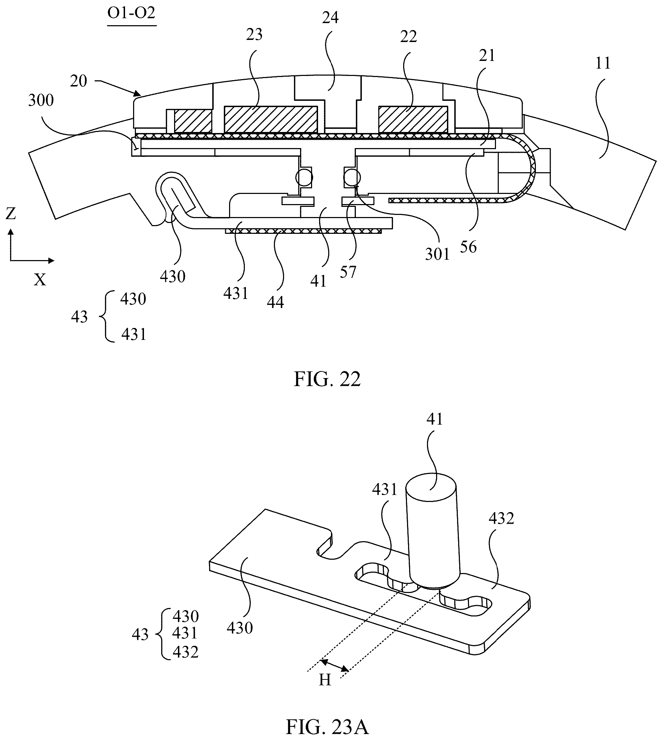

It can be learned from the foregoing that both the monitoring apparatus 20 shown in FIG. 16A and the monitoring apparatus 20 shown in FIG. 20 are described by using an example in which the monitoring apparatus 20 is the dual-channel monitoring structure. In some other embodiments of this application, as shown in FIG. 22 (a sectional view obtained by performing sectioning along a dashed line O1-O2 in FIG. 2B), the monitoring apparatus 20 may alternatively be a single-channel monitoring structure, and the pressure-sensitive element 44 is disposed in the middle frame 11. Specifically, the monitoring apparatus 20 includes the first circuit board 21, the light emitting device 22, the optical-to-electrical converter 23, the metal cover plate 24, the first lever 41, the deformation plate 43, the pressure-sensitive element 44, and the like. The first circuit board 21 may be an FPC. A disposition manner and a working process of the light emitting device 22, the optical-to-electrical converter 23, and the metal cover plate 24 are the same as those described above, and details are not described herein again.

Based on this, as shown in FIG. 22, the first mounting hole 301 that penetrates the middle frame 11 is disposed at the bottom of the mounting groove 300. The first lever 41 penetrates the first mounting hole 301, the first lever 41 is disposed on the side of the first circuit board that is away from the metal cover plate 24, and the first end of the first lever 41 abuts against or is connected to the first circuit board 21. The deformation plate 43 is located in the middle frame 11, and the deformation plate 43 is disposed on the side of the first lever 41 that is away from the first circuit board 21. The deformation plate 43 has the first cantilever end 431 and the fastening end 430, the first cantilever end 431 abuts against the second end of the first lever 41, and the fastening end 430 of the deformation plate 43 is connected to the middle frame 11. For example, a mounting hole may be disposed on an inner wall of the middle frame 11, the fastening end 430 of the deformation plate 43 may be embedded in the mounting hole, and the fastening end 430 of the deformation plate 43 is bonded to the middle frame 11 through an adhesive layer. Alternatively, a threaded connector may be used to penetrate the fastening end 430 of the deformation plate 43 and connect to the middle frame 11, to fasten the fastening end 430 of the deformation plate 43 to the middle frame 11.

In addition, the pressure-sensitive element 44 is located in the middle frame 11, is disposed on a side of the deformation plate 43 that is away from the metal cover plate 24, and the pressure-sensitive element 44 may cover at least a part of the first cantilever end 431 of the deformation plate 43. As described above, the pressure-sensitive element 44 is electrically connected to the first circuit board 21. The disposing manner and working process of the pressure-sensitive element 44 are the same as those described above, and are not described herein again. In addition, technical effect of disposing the pressure-sensitive element 44 in the middle frame 11 is the same as that described above, and is not described herein again. Based on this, in the structure shown in FIG. 22, the deformation plate 43 has only one cantilever end, that is, the first cantilever end 431. Therefore, a size of the deformation plate 43 can be reduced. This helps reduce a size of the entire monitoring apparatus 20.

On this basis, for example, the electronic device may include a first bonding layer 56 and a second clamping ring 57. The first bonding layer 56 is disposed between the first circuit board 21 and the bottom of the mounting groove 300, and the first bonding layer 56 may be connected to the first circuit board 21 and the bottom of the mounting groove 300. The first bonding layer 56 may be an adhesive layer, so that the first circuit board 21 and each component disposed on the first circuit board 21 can be fastened to the middle frame 11. In addition, the second clamping ring 57 is located in the middle frame 11, the second clamping ring 57 is clamped to the second end of the first lever 41, and the second clamping ring 57 abuts against the middle frame 11. In this way, in a process in which a user presses the monitoring apparatus 20, the second clamping ring 57 may limit movement of the first lever 41 in the Y direction.

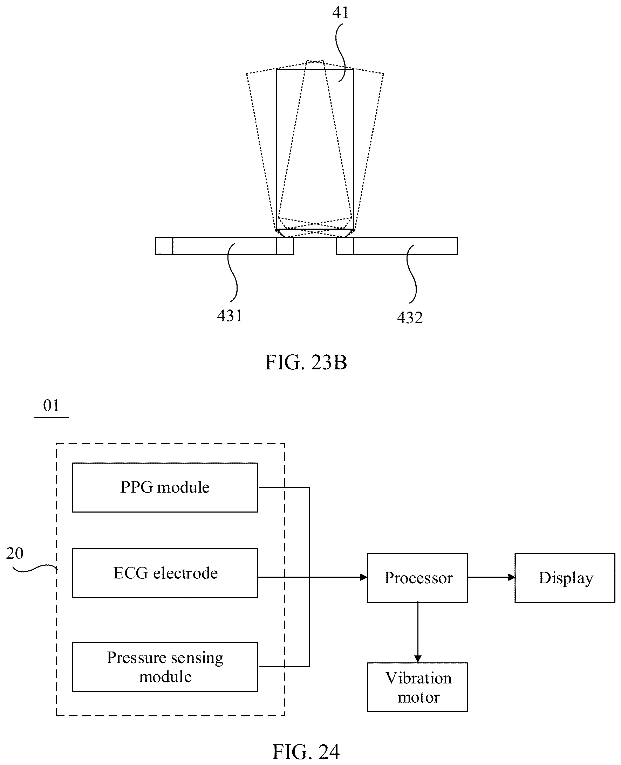

In some other embodiments of this application, when the monitoring apparatus 20 uses the structure shown in FIG. 22, the structure of the cantilever end of the deformation plate 43 may be changed, so that the monitoring apparatus may be the dual-channel monitoring structure. Specifically, as shown in FIG. 23A, the deformation plate 43 may include two cantilever ends, that is, the first cantilever end 431 and the second cantilever end 432. The second cantilever end 432 is located on a side that is of the first cantilever end 431 and that is away from the fastening end 430. There is a gap H between the first cantilever end 431 and the second cantilever end 432. The pressure-sensitive element 44 (as shown in FIG. 22) covers at least a part of the first cantilever end 431, and also covers at least a part of the second cantilever end 432. In addition, as shown in FIG. 23B, the second end (for example, the lower end) of the first lever 41 may abut against or be connected to both the first cantilever end 431 and the second cantilever end 432. In this way, one lever, that is, the first lever 41, may be disposed, so that the first cantilever end 431 and the second cantilever end 432 can be pressed at the same time, to drive the first cantilever end 431 and the second cantilever end 432 to deform at the same time, to implement dual-channel pressure monitoring.

In conclusion, as shown in FIG. 24, the monitoring apparatus 20 in the electronic device 01 provided in this embodiment of this application may include the PPG module, the ECG electrode, and the pressure sensing module. The PPG module (including the light emitting device 22 and the optical-to-electrical converter 23 shown in FIG. 16A) may obtain the PPG data such as the blood oxygen and the heart rate of the user. The ECG electrode, for example, the metal cover plate 24 or the metal cover plate 24 and the metal coating 241, may obtain the ECG data. The pressure sensing module includes components such as the first lever 41, the deformation plate 43, and the pressure-sensitive element 44, and is configured to monitor pressing pressure of the user. The pressure sensing module may be resistive, capacitive, or inductive. Still as shown in FIG. 24, the processor in the electronic device 01 may calculate a blood pressure value of the user based on the PPG data, the ECG data, and the detected pressure value, and then display the blood pressure, the blood oxygen, and heart rate information on the display.

On this basis, in some embodiments of this application, the monitoring apparatus 20 may further be used as a virtual button. For example, when the user presses the monitoring apparatus 20, after the processor obtains pressing pressure of the user through the pressure sensing module, the processor may control a vibration motor to vibrate, and a vibration force may match the pressing pressure of the user, so that the user can perceive a pressing experience. In addition, the processor may further control the electronic device 01 to enable a corresponding button function, to implement a function of the virtual button.

Alternatively, in some other embodiments of this application, the monitoring apparatus 20 may further be used as a part of a physical button. In this case, the electronic device may further include a meta dome connected to the pressure sensing module in the monitoring apparatus 20. When the user presses the monitoring apparatus 20, the meta dome may be driven to deform, to perform the button function.

The foregoing is an example for description by using an example in which the pressure sensing module is integrated into the monitoring apparatus 20. In some other embodiments of this application, the monitoring apparatus 20 having the PPG module and the ECG electrode may be disposed separately from the pressure sensing module. In this case, the monitoring apparatus 20 may not have the button function.

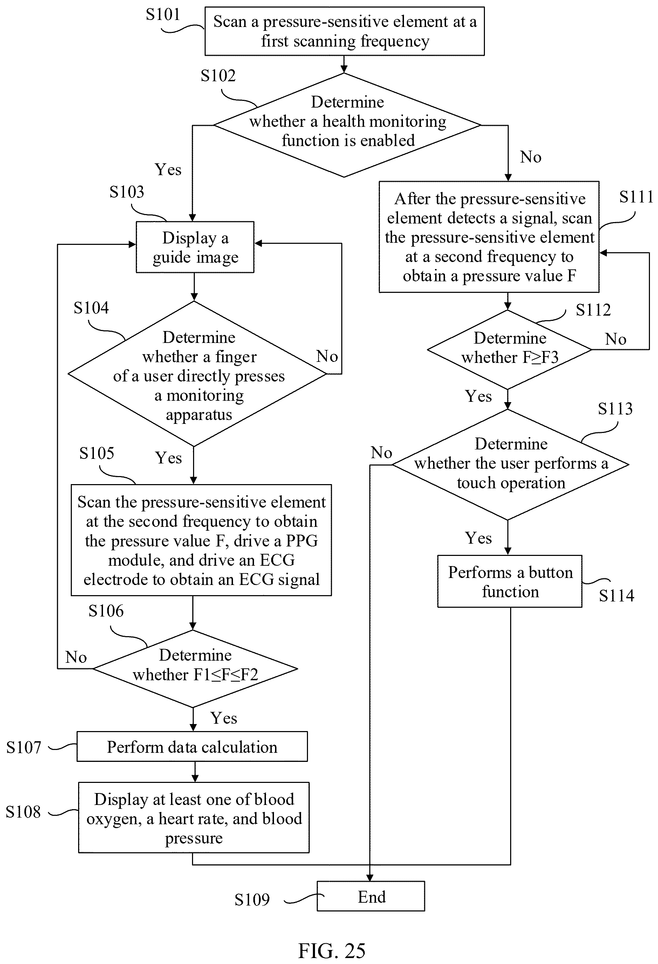

The following describes, by using an example in which the monitoring apparatus 20 has the button function and the health monitoring function, a control method for the electronic device 01 having the monitoring apparatus 20. Specifically, as shown in FIG. 25, the control method for the electronic device 01 may include S101 to S114.

S101: Scan a pressure-sensitive element at a first scanning frequency.

For example, a pressing event of the user on the monitoring apparatus 20 may be identified by performing S101. In addition, the pressure-sensitive element 44 in the pressure sensing module 400 is scanned at the first scanning frequency f1 with a low frequency, so that the pressure sensing module 400 runs at low first power consumption, to reduce power consumption.

S102: Determine whether a health monitoring function is enabled.

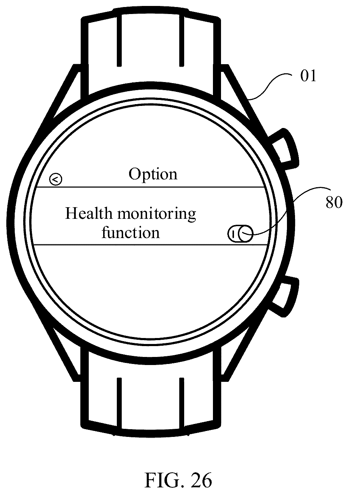

For example, as shown in FIG. 26, a health monitoring function button 80 may be displayed on a display of the electronic device 01. The user touches the health monitoring function button 80 on the display to enable or disable the health monitoring function. The health monitoring function may include monitoring data such as the blood oxygen, the heart rate, or the blood pressure of the user. Therefore, determining whether the health monitoring function is enabled is specifically monitoring a function enabling operation of the light emitting device 22 and a function enabling operation of the optical-to-electrical converter 23 in the monitoring apparatus 20. When the health monitoring function is enabled, the processor may monitor the function enabling operation of the light emitting device 22 and the function enabling operation of the optical-to-electrical converter 23. When the health monitoring function is disabled, the processor cannot monitor the function enabling operation of the light emitting device 22 and the function enabling operation of the optical-to-electrical converter 23.

Based on this, when a determining result of S102 shown in FIG. 25 is "no", that is, when the health monitoring function is disabled, the following S111 to S114 may be performed to perform the button function. Alternatively, when a determining result of S102 is "yes", that is, when the health monitoring function is enabled, the following S103 to S108 may be performed to perform the health monitoring function.

S103: Display a guide image.

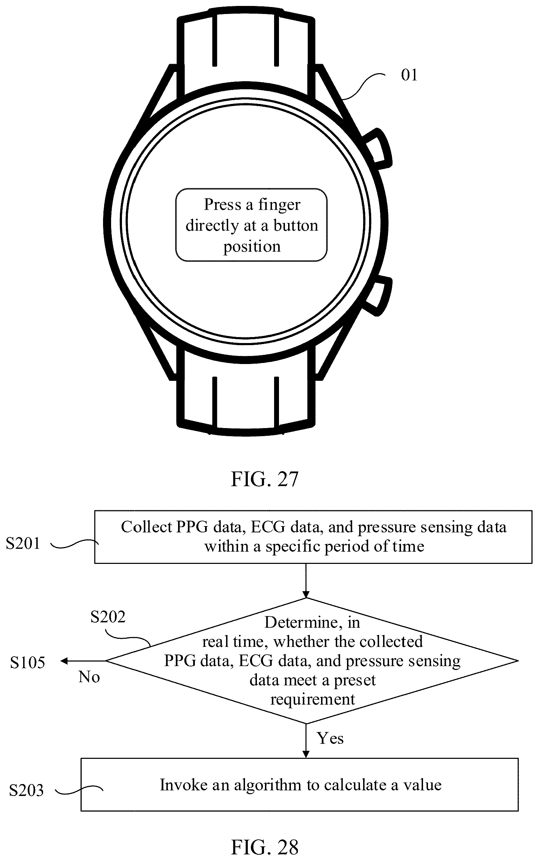

In some embodiments of this application, the electronic device 01 may display the guide image. The guide image is used to display a guide message in a health monitoring function execution process, to prompt the user to complete health monitoring. For example, when health monitoring starts, prompt information "Please press a finger directly at a button position" shown in FIG. 27 may be displayed. In the health monitoring process, the guide image may be adjusted based on content that needs to be guided. Content of the guide image is not limited in this application.

S104: Determine whether a human body (for example, a finger) of the user directly presses the monitoring apparatus.

For example, in some embodiments, because the health monitoring function is enabled, when the finger of the user directly presses the monitoring apparatus 20, infrared proximity minitoring may be implemented by using the infrared light when the light emitting device 22 in the monitoring apparatus 20 shown in FIG. 16A emits the infrared light. Because the infrared light can be absorbed by hemoglobin and deoxygenated hemoglobin in blood, the finger of the user directly pressing the monitoring apparatus 20 can be determined by obtaining the PPG data.

Alternatively, in some other embodiments, a voltage may be applied to the metal cover plate 24, so that the metal cover plate 24 is used as the ECG electrode. When the finger of the user presses the monitoring apparatus 20, whether the finger of the user directly presses the monitoring apparatus 20 may be determined by obtaining a change of impedance on a path formed by the ECG electrode and the human body. Based on this, when a determining result of S104 is "yes", the following S105 may be performed. When a determining result of S104 is "no", S103 may be performed to display the guide image shown in FIG. 27.

S105: Scan the pressure-sensitive element at a second frequency to obtain a pressure value F, drive the PPG module, and drive the ECG electrode to obtain an electrocardiosignal.

For example, the processor may scan the pressure-sensitive element 44 in the pressure sensing module 400 at a high second scanning frequency f2 (where f2>f1), so that the pressure sensing module 400 runs at high second power consumption (the second power consumption is greater than the first power consumption), to obtain the pressure value F applied by the user to the metal cover plate 24. The pressure sensing module 400 (as shown in FIG. 13) in the monitoring apparatus 20 may use the foregoing resistive, capacitive, or inductive pressure sensing monitoring principle.

In addition, the processor may drive all light emitting elements (including a visible light emitting element and an infrared light emitting element) in the light emitting device 22 and the optical-to-electrical converter 23 in the monitoring apparatus 20 to work, to start the PPG module, and obtain the PPG data (including blood oxygen data and heart rate data) of the user by obtaining the output signal of the optical-to-electrical converter 23. In addition, the processor may further apply a voltage to the metal cover plate 24, to drive the metal cover plate 24 to be used as the ECG electrode, to obtain the electrocardiosignal.

S106: Determine whether F1≤F≤F2.

For example, in a process in which the processor performs S106 shown in FIG. 25, if the pressure value F obtained in S102 is greater than or equal to a first threshold F1 and less than or equal to a second threshold F2, the following S107 may be performed. The second threshold F2 is greater than the first threshold F1. For example, F1=10 gf, and F2=300 gf.

S107: Perform data calculation. For example, S108 may include S201 to S203 shown in FIG. 28.

S201: Collect PPG data, ECG data, and pressure sensing data within a specific period of time.

For example, to improve precision of calculating blood pressure, the processor may continuously collect the PPG data, the ECG data, and the pressure sensing data within a specific period of time.

S202: Determine, in real time, whether the collected PPG data, ECG data, and pressure sensing data meet a preset requirement.

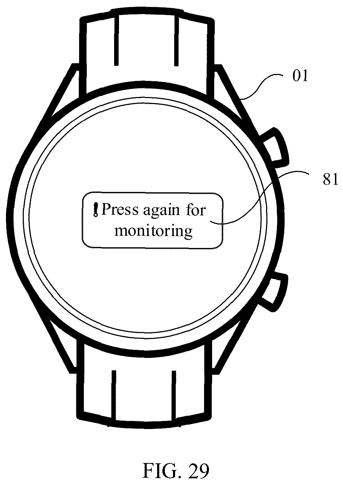

For example, the processor may compare the collected PPG data, ECG data, and pressure sensing data with the preset requirement, for example, data precision and data duration, in real time. If a determining result of S202 is "yes", that is, the foregoing data meets the preset requirement, S203 shown in FIG. 25 is performed. If a determining result of S202 is "no", that is, the foregoing data does not meet the preset requirement, for example, the finger of the user does not keep in a pressing state, a prompt dialog box 81 shown in FIG. 29 may be displayed, to prompt the user to press the monitoring apparatus 20 again. After the user presses the monitoring apparatus again, S105 shown in FIG. 25 may be performed to obtain the pressure value F again.

S203: Invoke an algorithm to calculate a value. For example, the processor may invoke a related algorithm from a memory, and determine a monitoring result based on at least one of the pressure value, the output signal of the optical-to-electrical converter, and the electrocardiosignal obtained in S107 shown in FIG. 25. The monitoring result may include at least one of the blood oxygen value, the heart rate value, the electrocardiogram data, and the blood pressure value.

S108: Display at least one of the blood oxygen, the heart rate, and the blood pressure.

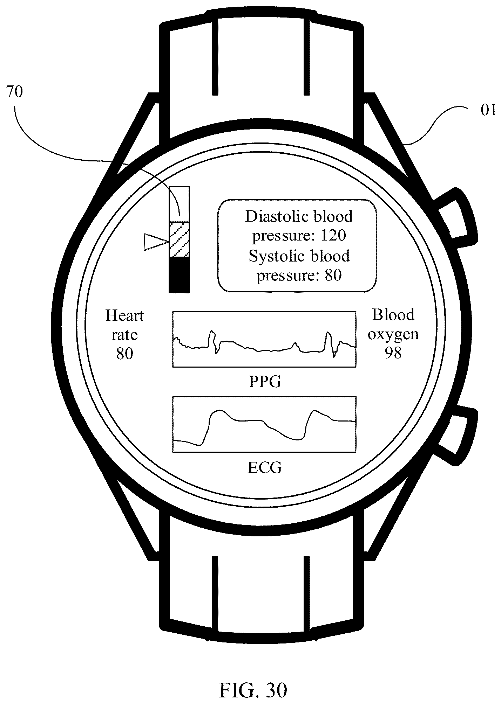

As shown in FIG. 30, the electronic device 01 may display a monitoring result. The monitoring result includes at least one of the foregoing blood oxygen, heart rate, and blood pressure data. In addition, the foregoing blood oxygen, heart rate, and blood pressure data may change in real time based on a change of pressing strength of the user. When pressing strength of the user reaches a middle stage of a pressure scale 70, the displayed blood oxygen, heart rate, and blood pressure data are used as final monitoring data.

An arrow in the pressure scale 70 may move on the scale with the pressing force of the user. Stages of different colors on the pressure scale 70 may indicate a magnitude of the pressing force of the user. Alternatively, in some other embodiments, a magnitude of a pressure value F, for example, 200 gf, may be further displayed on one side of the pressure scale 70. In this way, the user may be guided to apply a pressure value within a preset range, for example, (10 gf to 300 gf), and the range may correspond to a position in the middle stage of the pressure scale 70, to improve monitoring precision. Alternatively, when the monitoring apparatus 20 has a dual-channel pressure sensing monitoring function, the electronic device 01 may further display a pressing position prompt image based on a result of dual-channel pressure sensing monitoring, to prompt the user to press the electronic device 01 to a preset position, to improve monitoring precision.

S109: End.

In addition, it can be learned from the foregoing that when a determining result of S102 shown in FIG. 25 is "no", that is, when the health monitoring function is disabled, the following S111 to S114 may be performed to perform the button function.

S111: After detecting a signal, the pressure-sensitive element scans the pressure-sensitive element at the second scanning frequency to obtain the pressure value F.

The signal detected by the pressure-sensitive element 44 is a pressure signal generated when the user presses the metal cover plate 24 of the monitoring apparatus 20.

S112: Determine whether F≥F3.

For example, if the pressure value F obtained in S112 is greater than or equal to the third threshold F3, S113 is performed. The third threshold F3 may be within a range of 10 gf to 300 gf. When a determining result of S112 is "no", S111 is performed to obtain the pressure value F again.

S113: Determine whether the user performs a touch operation.

For example, to avoid that the monitoring apparatus 20 is mistakenly triggered due to a "non-user trigger reason" such as a "temperature conflict" caused by an instantaneous abrupt change of an ambient temperature of the electronic device when the user uses the electronic device, in some embodiments of this application, before S114 is performed, S113 may be performed to determine whether the pressing force applied to the monitoring apparatus 20 is from the touch operation of the user. Specifically, when the light emitting device 22 in the monitoring apparatus 20 shown in FIG. 16A can emit the infrared light, infrared proximity minitoring may be implemented by using the infrared light. If the touch operation of the user on the metal cover plate 24 is detected, S114 may be performed. If the touch operation of the user on the metal cover plate 24 is not detected, S109 may be performed. In some other embodiments of this application, S113 may not need to be set in the foregoing control method.

S114: Perform the button function.

For example, in a process of performing S114, when the monitoring apparatus 20 is the virtual button, the processor may control the vibration motor shown in FIG. 24 to work, and perform the button function. Alternatively, when the monitoring apparatus 20 is the physical button, the monitoring apparatus 20 may drive the meta dome to deform, and perform the button function.

After S114 is performed, S109 may be performed to end the control method.

In conclusion, according to the control method for the electronic device 01 provided in this embodiment of this application, both health data monitoring and a button response function can be implemented. Therefore, health monitoring and the button may be integrated into a same monitoring apparatus 20, to improve integration of the electronic device 01 and reduce a size of the electronic device 01.

The foregoing descriptions are merely specific implementations of this application, but are not intended to limit the protection scope of this application. Any variation or replacement within the technical scope disclosed in this application shall fall within the protection scope of this application. Therefore, the protection scope of this application shall be subject to the protection scope of the claims.

## Claims

1. A monitoring apparatus, comprising:
a first circuit board;
a light emitting device, disposed on the first circuit board, and electrically connected to the first circuit board;
an optical-to-electrical converter, wherein the optical-to-electrical converter and the light emitting device are disposed on a same side of the first circuit board, and the optical-to-electrical converter is electrically connected to the first circuit board;
a metal cover plate, covering the first circuit board, wherein the metal cover plate is electrically connected to the first circuit board, an accommodation cavity is formed between the metal cover plate and the first circuit board, the light emitting device and the optical-to-electrical converter are disposed in the accommodation cavity, a first light through hole and a second light through hole that penetrate the metal cover plate are disposed on the metal cover plate, the first light through hole exposes a light exit surface of the light emitting device, and the second light through hole exposes a light receiving surface of the optical-to-electrical converter; and
a light transmission structure, filling the first light through hole, the second light through hole, and the accommodation cavity, wherein the light transmission structure wraps the light emitting device and the optical-to-electrical converter, and is connected to the light emitting device, the optical-to-electrical converter, the first circuit board, and the metal cover plate.

2. The monitoring apparatus according to claim 1, wherein the monitoring apparatus comprises:
a first lever, disposed on a side that is of the first circuit board and that is away from the metal cover plate, wherein a first end of the first lever abuts against or is connected to the first circuit board;
a deformation plate, disposed on a side that is of the first lever and that is away from the first circuit board, wherein the deformation plate has a first cantilever end and a fastening end, the first cantilever end abuts against or is connected to a second end of the first lever, and the fastening end is configured to fasten the deformation plate; and
a pressure-sensitive element, covering at least a part of the first cantilever end, wherein the pressure-sensitive element is electrically connected to the first circuit board.

3. The monitoring apparatus according to claim 2, wherein
the pressure-sensitive element is disposed on a side that is of the deformation plate and that faces the metal cover plate, and the monitoring apparatus further comprises:
a mounting pillar, located on a side that is of the deformation plate and that is away from the metal cover plate, wherein a first end of the mounting pillar is connected to the fastening end, and a second end of the mounting pillar is configured to fasten the monitoring apparatus.

4. The monitoring apparatus according to claim 2 or 3, wherein
the pressure-sensitive element comprises a resistance strain gauge, and a part that is of the resistance strain gauge and that covers the first cantilever end has a first resistor and a second resistor; and
the monitoring apparatus further comprises a third resistor and a fourth resistor, wherein the first resistor and the second resistor are connected in series to form a first circuit, the third resistor and the fourth resistor are connected in series to form a second circuit, the first circuit and the second circuit are connected in parallel to form a first full-bridge circuit, and the first full-bridge circuit is electrically connected to the first circuit board.

5. The monitoring apparatus according to claim 4, wherein
the deformation plate further has a second cantilever end, the fastening end is located between the first cantilever end and the second cantilever end, and the pressure-sensitive element covers at least a part of the second cantilever end; and
the monitoring apparatus further comprises a second lever, the second lever is disposed on the side that is of the first circuit board and that is away from the metal cover plate, a first end of the second lever abuts against or is connected to the first circuit board, and a second end of the second lever abuts against or is connected to the second cantilever end.

6. The monitoring apparatus according to claim 4, wherein
the deformation plate further has a second cantilever end, the second cantilever end is located on a side that is of the first cantilever end and that is away from the fastening end, there is a gap between the first cantilever end and the second cantilever end, and the pressure-sensitive element covers at least a part of the second cantilever end; and
the second end of the first lever further abuts against or is connected to the second cantilever end.

7. The monitoring apparatus according to claim 5 or 6, wherein a part that is of the resistance strain gauge and that covers the second cantilever end has a fifth resistor and a sixth resistor, the fifth resistor and the sixth resistor are connected in series to form a third circuit, the second circuit and the third circuit are connected in parallel to form a second full-bridge circuit, and the second full-bridge circuit is electrically connected to the first circuit board.

8. The monitoring apparatus according to claim 2, 5, or 6, wherein
the first circuit board comprises:
a flexible circuit board; and
a supportive plate, disposed on a side that is of the flexible circuit board and that is away from the metal cover plate, wherein the supportive plate is connected to or abuts against the first lever;
or
the first circuit board comprises a printed circuit board.

9. The monitoring apparatus according to claim 8, wherein the monitoring apparatus further comprises:
a first metal electrode plate, disposed on a side that is of the supportive plate and that is away from the metal cover plate, wherein the first metal electrode plate is electrically connected to the flexible circuit board; and
the pressure-sensitive element is located between the deformation plate and the metal supportive plate, the pressure-sensitive element comprises a second metal electrode plate, and a capacitor is formed between the second metal electrode plate and the first metal electrode plate.

10. The monitoring apparatus according to claim 8, wherein
the supportive plate comprises a metal material; and
the pressure-sensitive element is located between the deformation plate and the supportive plate, and the pressure-sensitive element comprises a metal coil.

11. The monitoring apparatus according to any one of claims 1 to 10, wherein the monitoring apparatus further comprises:
conductive silicone gel, disposed between the metal cover plate and the first circuit board, wherein a first end of the conductive silicone gel is electrically connected to the metal cover plate, and a second end of the conductive silicone gel is electrically connected to the first circuit board.

12. The monitoring apparatus according to any one of claims 1 to 10, wherein the monitoring apparatus further comprises:
conductive adhesive, disposed between the metal cover plate and the first circuit board, wherein the conductive adhesive is bonded to the metal cover plate and a copper exposure part of the first circuit board, and the conductive adhesive is electrically connected to the metal cover plate and the first circuit board.

13. The monitoring apparatus according to any one of claims 1 to 10, wherein
the monitoring apparatus further comprises:
a metal dome, disposed in the accommodation cavity, wherein a first end of the metal dome is welded to the metal cover plate, and a second end of the metal dome abuts against a copper exposure part of the first circuit board; or a second end of the metal dome is welded to the first circuit board, and a first end of the metal dome abuts against the metal cover plate.

14. The monitoring apparatus according to any one of claims 1 to 13, wherein the metal cover plate comprises:
a metal cover plate body, covering the first circuit board, wherein the metal cover plate body is electrically connected to the first circuit board, and the first light through hole and the second light through hole that are spaced from each other are disposed on the metal cover plate body; and
a light shielding blocking plate, disposed in the accommodation cavity, wherein the light shielding blocking plate is connected to the metal cover plate body and the first circuit board, and the light shielding blocking plate is located between the first light through hole and the second light through hole, and separates the light emitting device from the optical-to-electrical converter.

15. The monitoring apparatus according to any one of claims 1 to 14, wherein
a light shielding connection layer is disposed between the metal cover plate and the first circuit board, and the light shielding connection layer is connected to the metal cover plate and the first circuit board.

16. The monitoring apparatus according to claim 15, wherein a surface that is of the light transmission structure and that is away from the first circuit board and a surface that is of the metal cover plate and that is away from the first circuit board are spliced into an arc surface.

17. The monitoring apparatus according to any one of claims 1 to 16, wherein the monitoring apparatus comprises a metal coating, and the metal coating covers the surface that is of the metal cover plate and that is away from the first circuit board.

18. The monitoring apparatus according to any one of claims 1 to 17, wherein the light emitting device comprises:
a first light emitting component, configured to emit at least one of red light or infrared light, wherein there is a first spacing d1 between the first light emitting component and the optical-to-electrical converter; and
a second light emitting component, configured to emit at least one of yellow light, green light, or blue light, wherein there is a second spacing d2 between the second light emitting component and the optical-to-electrical converter, and d1>d2.

19. An electronic device, comprising:
the monitoring apparatus according to any one of claims 1 to 18; and
a middle frame, wherein the monitoring apparatus is connected to the middle frame, a mounting groove is disposed on an outer surface of the middle frame; and the first circuit board, the light emitting device, the optical-to-electrical converter, the metal cover plate, and the light transmission structure are located in the mounting groove, and the first circuit board is close to a bottom of the mounting groove relative to the metal cover plate.

20. The electronic device according to claim 19, wherein a first mounting hole that penetrates the middle frame is disposed at the bottom of the mounting groove, and the monitoring apparatus comprises:
a deformation plate, located in the mounting groove, wherein the deformation plate has a fastening end;
a mounting pillar, located on a side that is of the deformation plate and that is away from the metal cover plate, and penetrating the first mounting hole, wherein a first end of the mounting pillar is connected to the fastening end, and a second end of the mounting pillar extends out of the first mounting hole and is located in the middle frame; and
the electronic device further comprises:
an elastic layer, located in the mounting groove, wherein the elastic layer is disposed between the deformation plate and the bottom of the mounting groove; and
a first clamping ring, located in the middle frame, wherein the first clamping ring is clamped to the second end of the mounting pillar, and the first clamping ring abuts against the middle frame.

21. The electronic device according to claim 20, wherein
the elastic layer comprises silicone gel or rubber;
or
the elastic layer comprises a soft rubber layer and a plurality of particles doped in the soft rubber layer, hardness of the particle is greater than hardness of the soft rubber layer, the soft rubber layer comprises silicone gel or rubber, and the particle comprises at least one of metal, plastic, or rubber.

22. The electronic device according to claim 20 or 21, wherein
a first sealing groove is disposed on a part that is of the mounting pillar and that is located in the first mounting hole; and
the electronic device comprises a first sealing ring, the first sealing ring is located in the first sealing groove, and the first sealing ring abuts against a hole wall of the first mounting hole and a groove wall of the first sealing groove.

23. The electronic device according to claim 19, wherein
a first mounting hole that penetrates the middle frame is disposed on the bottom of the mounting groove, and the monitoring apparatus comprises:
a first lever, penetrating the first mounting hole, wherein the first lever is disposed on a side that is of the first circuit board and that is away from the metal cover plate, a first end of the first lever abuts against or is connected to the first circuit board, and a second end of the first lever extends out of the first mounting hole and is located in the middle frame; and
the electronic device further comprises:
a first bonding layer, located in the mounting groove, wherein the first bonding layer is disposed between the first circuit board and the bottom of the mounting groove, and is connected to the first circuit board and the bottom of the mounting groove; and
a second clamping ring, located in the middle frame, wherein the second clamping ring is clamped to the second end of the first lever, and the second clamping ring abuts against the middle frame.

24. The electronic device according to claim 19, wherein the monitoring apparatus further comprises:
a deformation plate, located in the middle frame, wherein the deformation plate has a fastening end; and
the electronic device further comprises a threaded connector, wherein the threaded connector is located in the middle frame, the threaded connector penetrates the fastening end of the deformation plate, and is connected to the middle frame.

25. The electronic device according to claim 19, wherein the electronic device further comprises:
a first mounting hole and a second mounting hole that penetrate the middle frame and that are disposed on the bottom of the mounting groove, and the monitoring apparatus comprises:
a first lever, penetrating the first mounting hole, wherein the first lever is disposed on a side that is of the first circuit board and that is away from the metal cover plate, a first end of the first lever abuts against or is connected to the first circuit board, and a second end of the first lever extends out of the first mounting hole and is located in the middle frame;
a second lever, penetrating the second mounting hole, wherein the second lever is disposed on the side that is of the first circuit board and that is away from the metal cover plate, a first end of the second lever abuts against or is connected to the first circuit board, and a second end of the second lever extends out of the second mounting hole and is located in the middle frame;
a first spring, located in the mounting groove, wherein a part of the first lever penetrates the first spring, and two ends of the first spring abut against the first lever and a groove bottom of the mounting groove respectively; and
a second spring, located in the mounting groove, wherein a part of the second lever penetrates the second spring, and two ends of the second spring abut against the second lever and the groove bottom of the mounting groove respectively.

26. The electronic device according to any one of claims 19 to 25, wherein the first circuit board comprises a flexible circuit board, and a third mounting hole that penetrates the middle frame is disposed on the bottom of the mounting groove; and
the electronic device further comprises:
a cap, disposed in the third mounting hole, and blocking the third mounting hole;
a second bonding layer, disposed in the third mounting hole, and located on a side that is of the cap and that is away from the mounting groove, wherein the second bonding layer is bonded to the cap and the middle frame; and
a second circuit board, disposed in the middle frame, wherein a part of the flexible circuit board penetrates the cap and the second bonding layer, extends into the middle frame, and is electrically connected to the second circuit board.

27. A control method of an electronic device, wherein the electronic device comprises a monitoring apparatus, the monitoring apparatus has a button function and a health monitoring function, and the monitoring apparatus comprises a light emitting device, an optical-to-electrical converter, a metal cover plate, and a pressure-sensitive element; and
the control method comprises:
scanning the pressure-sensitive element at a first scanning frequency to identify a pressing event of a user on the monitoring apparatus;
determining whether the health monitoring function is enabled; and
if the health monitoring function is enabled, performing the health monitoring function; or
if the health monitoring function is not enabled, performing the button function.

28. The control method according to claim 27, wherein the health monitoring function is specifically:
displaying a guide image;
if a human body of the user directly presses the monitoring apparatus, scanning the pressure-sensitive element at a second scanning frequency to obtain a pressure value applied by the user to the metal cover plate, starting the light emitting device and the optical-to-electrical converter, and applying a voltage to the metal cover plate to obtain an electrocardiosignal of the user, wherein the second scanning frequency is greater than the first scanning frequency;
if the pressure value is greater than or equal to a first threshold and less than or equal to a second threshold, determining a monitoring result based on at least one of the pressure value, an output signal of the optical-to-electrical converter, and the electrocardiosignal; and
displaying the monitoring result, wherein the monitoring result comprises at least one of a blood oxygen value, a heart rate value, electrocardiogram data, and a blood pressure value.

29. A computer storage medium, comprising computer instructions, wherein when the computer instructions are run on an electronic device, the electronic device is enabled to perform the control method of the electronic device according to claim 27 or 28.

30. A computer program product, wherein when the computer program product runs on a computer, the computer is enabled to perform the control method of the electronic device according to claim 27 or 28.
